# EUROPÄISCHE PATENTSCHRIFT

(11) **EP 1 503 995 B1**
(45) Veröffentlichungstag und Bekanntmachung des Hinweises auf die Patenterteilung: **12.12.2007**
(21) Anmeldenummer: 03725046.1
(22) Anmeldetag: 16.04.2003
(51) Int. Cl.: C07D 251/42

(54) **VERFAHREN ZUR HERSTELLUNG VON SUBSTITUIERTEN PHENYLSULFONYLHARNSTOFFEN**
METHOD FOR PRODUCING SUBSTITUTED PHENYLSULFONYL UREA
PROCEDE DE PRODUCTION DES UREES DE PHENYLSULFONYLE SUBSTITUEES

(30) Priorität: 26.04.2002 DE 10218704
(43) Veröffentlichungstag der Anmeldung: 09.02.2005
(73) Patentinhaber: Bayer CropScience AG, 40789 Monheim (DE)
(72) Erfinder: VERMEHREN, Jan, 65510 Idstein (DE); FORD, Mark, James, 65815 Bad Soden (DE); SCHLEGEL, Guenter, 51381 Leverkusen (DE); KUEBEL, Boerries, 65779 Kelkheim (DE); ORT, Oswald, 61479 Glashütten (DE)
(86) Internationale Anmeldenummer: PCT/EP2003/003980
(87) Internationale Veröffentlichungsnummer: WO 2003/091228

(56) Entgegenhaltungen:
- EP-A- 0 378 082
- WO-A-92/13845
- DE-A- 2 616 612
- DE-A- 19 946 341
- DATABASE CROSSFIRE BEILSTEIN [Online] Beilstein Institut zur Förderung der Chemischen Wissenschaften, Frankfurt am Main, DE; XP002253556 & FIELDING ET AL.: J. FLUORINE CHEM., Bd. 59, Nr. 1, 1992, Seiten 15-31,
- DATABASE CROSSFIRE BEILSTEIN [Online] Beilstein Institut zur Förderung der Chemischen Wissenschaften, Frankfurt am Main, DE; XP002253557 & BLANCHARD: AM. CHEM. J., Bd. 30, 1903, Seite 508

## Beschreibung

Halosulfonylbenzoesäurehalogenide, Verfahren zu deren Herstellung und deren Verwendung zur Herstellung von substituierten Phenylsulfonylharnstoffen

Die Erfindung betrifft das technische Gebiet der chemischen Verfahren zur Herstellung von Verbindungen aus der Reihe herbizider Phenylsulfonylharnstoffe und deren Zwischenprodukte.

Eine Reihe von substituierten Phenylsulfonylharnstoffen sind als Herbizide und Pflanzenwachstumsregulatoren beschrieben worden. Innerhalb der Gruppe der Phenylsulfonylharnstoffe werfen solche mit einer Carboxygruppe oder einer Carbonsäurederivatgruppe am Phenylring besondere synthetische Fragen auf. Von Interesse sind die aus EP-A-007687 oder WO-A-92/13845 bekannten Verbindungen der Formel (I) und deren Salze, 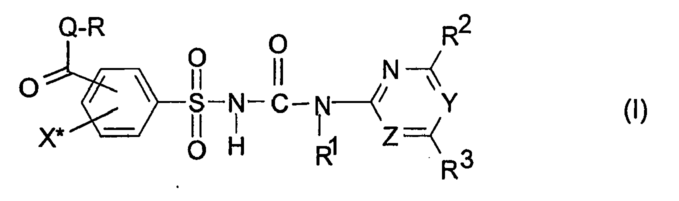 worin
- Q: Sauerstoff oder Schwefel,
- X*: Wasserstoff, Halogen, Cyano, Nitro, (C₁-C₃)-Alkyl oder Methoxy, vorzugsweise Wasserstoff oder Iod, insbesondere Iod,
- Y,Z: unabhängig voneinander CH oder N, wobei Y und Z nicht gleichzeitig CH sind,
- R: Wasserstoff, (C₁-C₁₂)-Alkyl, (C₂-C₁₀)-Alkenyl, (C₂-C₁₀)-Alkinyl, (C₁-C₆)-Alkyl, das ein- bis vierfach durch Reste aus der Gruppe Halogen, (C₁-C₄)Alkoxy, (C₁-C₄)-Alkylthio, CN, [(C₁-C₄)-Alkoxy]-carbonyl und (C₂-C₆)-Alkenyl substituiert ist, oder (C₃-C₈)-Cycloalkyl, das unsubstituiert oder durch Reste aus der Gruppe (C₁-C₄)-Alkyl, (C₁-C₄)-Alkoxy, (C₁-C₄)-Alkylthio und Halogen substituiert ist, (C₅-C₈)-Cycloalkenyl, Phenyl-(C₁-C₄)-alkyl, das im Phenylrest unsubstituiert oder durch einen oder mehrere Reste aus der Gruppe Halogen, (C₁-C₄)-Alkyl, (C₁-C₄)-Alkoxy, (C₁-C₄)-Haloalkyl, (C₁-C₄)-Alkylthio, [(C₁-C₄)-Alkoxy]-carbonyl, [(C₁-C₄)-Alkyl]-carbonyloxy, Carbamoyl, [(C₁-C₄)-Alkyl]-carbonylamino, [(C₁-C₄)-Alkyl]-aminocarbonyl, Di-[(C₁-C₄)-alkyl]-aminocarbonyl und Nitro substituiert ist, oder einen Rest der Formeln A-1 bis A-10 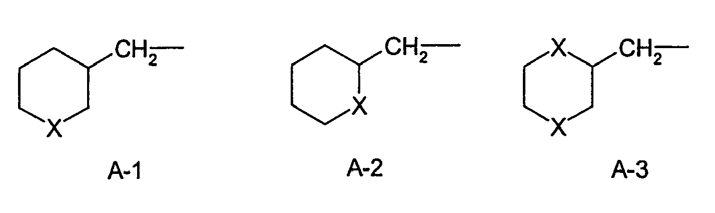 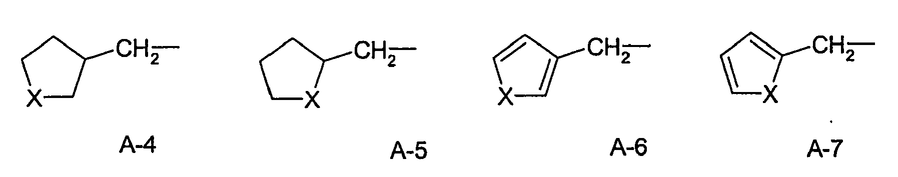 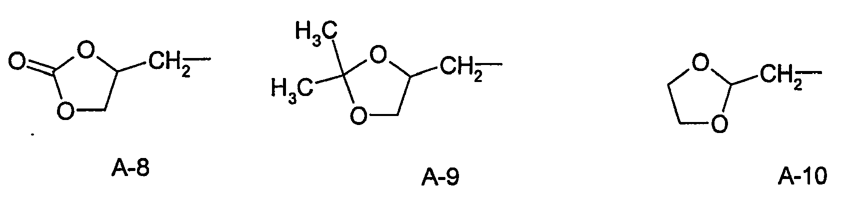 wobei in den Formeln A-1 bis A-10
X oder die X unabhängig voneinander O, S, S(O) oder SO₂ bedeuten,
- R¹: Wasserstoff oder (C₁-C₃)-Alkyl,
- R²: Wasserstoff, Halogen, (C₁-C₃)-Alkyl oder (C₁-C₃)-Alkoxy, wobei jeder der beiden letztgenannten Reste unsubstituiert oder ein- oder mehrfach durch Halogen oder (C₁-C₃)-Alkoxy substituiert ist,
- R³: Wasserstoff, Halogen, (C₁-C₃)-Alkyl, (C₁-C₃)-Alkoxy oder (C₁-C₃)-Alkylthio, wobei jeder der letztgenannten drei Reste unsubstituiert oder ein- oder mehrfach durch Halogen oder ein- oder zweifach durch (C₁-C₃)Alkoxy oder (C₁-C₃)-Alkylthlo substituiert ist, oder einen Rest der Formel NR⁴R⁵, (C₃-C₆)-Cycloalkyl, (C₂C₄)-Alkenyl, (C₂-C₄)-Alkinyl, (C₃-C₄)-Alkenyloxy oder (C₃-C₄)-Alkinyloxy,
- R⁴ und R⁵: unabhängig voneinander Wasserstoff, (C₁-C₄)-Alkyl, (C₃-C₄)-Alkenyl, (C₁-C₄)-Haloalkyl oder (C₁-C₄)-Alkoxy
bedeuten.

Die Salze der Verbindungen (I) sind vorzugsweise Verbindungen, in denen das Wasserstoffatom in der SO₂NH-Gruppe des Sulfonylharnstoffs durch ein Kation ersetzt ist, vorzugsweise ein physiologisch verträgliches und im Pflanzenschutz einsetzbares Kation, insbesondere ein Alkalimetall- oder Erdalkalimetallkation oder ein gegebenenfalls substituiertes Ammoniumion, inklusive quartäre Ammoniumionen. Beispiele für Kationen sind das Natrium-, Kalium- und Ammoniumion.
Salze der Verbindungen der Formel (I) können durch Anlagerung einer geeigneten anorganischen oder organischen Säure, wie beispielsweise HCI, HBr, H₂SO₄ oder HNO₃, aber auch Oxalsäure oder Sulfonsäuren an eine basische Gruppe, wie z.B. Amino oder Alkylamino, gebildet werden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Salze können ebenfalls dadurch gebildet werden, dass bei geeigneter funktioneller Gruppe, wie z.B. der Carboxygruppe, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quaternäre Ammoniumsalze.

Von besonderem Interesse sind Verbindungen der Formel (I) oder deren Salze bzw. deren Herstellung, worin die Gruppe der Formel -CO-Q-R in Orthostellung zur Sulfonylgruppe des Sulfonylharnstoffs (I) steht. Bevorzugt sind Verbindungen (I) oder deren Salze, worin Q = Sauerstoffatom, X* = Wasserstoff oder Halogen, vorzugsweise Iod, R = (C₁-C₄)-Alkyl, (C₂-C₄)-Alkenyl, (C₂-C₄)-Alkinyl, (C₁-C₄)-Haloalkyl, oder (C₁-C₄)-Alkoxy(C₁-C₄)-alkyl, vorzugsweise Methyl oder Ethyl, insbesondere Methyl bedeuten. Weiter bevorzugt sind Verbindungen (I) und deren Salze, worin die Gruppe der Formel -CO-Q-R in Orthostellung zur Sulfonylgruppe des Sulfonylharnstoffs, X* = Halogen, vorzugsweise Iod, und X* in Parastellung zur Gruppe der Formel -CO-Q-R steht. Weiter bevorzugt sind dabei und generell Verbindungen (I) und deren Salze, worin Z ein Stickstoffatom und Y ein Stickstoffatom oder eine Gruppe der Formel CH bedeuten, vorzugsweise Y ein Stickstoffatom bedeutet.

Aus WO-A-92/13845 und WO 01/23368 und dort zitierter Literatur ist bekannt, Phenylsulfohalogenide, die am Phenylring mit Carbonsäurederivatresten und zusätzlich anderen Resten wie Halogen substituiert sind, durch Ammonolyse zu Sulfonamiden umzusetzen, welche nach Phosgenierung zu Isocyanaten und weiterer Reaktion mit heterocyclischen Aminoverbindungen zu Sulfonylharnstoffen der Formel (I) oder deren Salzen umgesetzt werden.
Das Ausgangsmaterial (das betreffende Phenylsulfochlorid) ist gemäß WO 92/13845 aus substituierten Aminobenzoesäuren nach Veresterung, Diazotierung, Umsetzung mit SO₂ in Gegenwart von Cu-Katalysatoren (Meerwein-Reaktion) und oxidativer Spaltung des erhaltenen Disulfids mit Chlorgas in Salzsäure herstellbar.

Der bekannte Reaktionsweg ist jedoch von der Ausbeute und Zahl der Stufen nicht befriedigend. Insbesondere die Bereitstellung des multifunktionellen Phenylsulfochlorids ist aufwendig und von der Ausbeute her nicht optimal. Außerdem erfordert die anschließende Phosgenierung besondere, aufwendige Techniken zur Durchführung und Verfahrenskontrolle. Es besteht deshalb die Aufgabe ein alternatives Verfahren bereitzustellen, das im Vergleich zu den bekannten Verfahren hinsichtlich eines Aspekts, vorzugsweise mehrerer Aspekte vorteilhaft durchzuführen ist.

Gegenstand der Erfindung ist ein Verfahren zur Herstellung der genannten Phenylsulfonylharnstoffe der Formel (I) und deren Salzen,
dadurch gekennzeichnet, dass man
a) eine Verbindung der Formel (II) 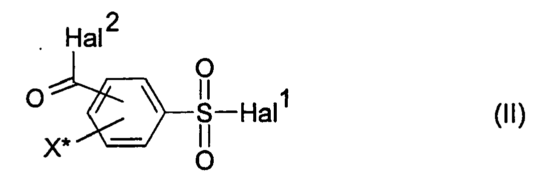 worin
   - Hal¹: ein Halogenatom, vorzugsweise Chlor oder Brom, insbesondere Chlor, bedeutet,
   - Hal²: ein Halogenatom, vorzugsweise Chlor oder Brom, insbesondere Chlor, bedeutet, und
   - X*: wie in Formel (I) definiert ist,
   durch Reaktion mit einer Verbindung der Formel R-Q-H oder einem Salz davon zur Verbindung der Formel (III) umsetzt, 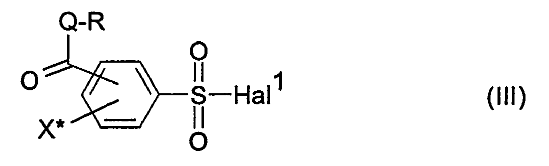 wobei R, Q und X wie in Formel (I) und Hal¹ wie in Formel (II) definiert sind, und
(b) mit oder ohne Zwischenisolierung entweder
   (b1) die erhaltene Verbindung (III) zum Sulfonamid der Formel (IV) ammonolysiert, 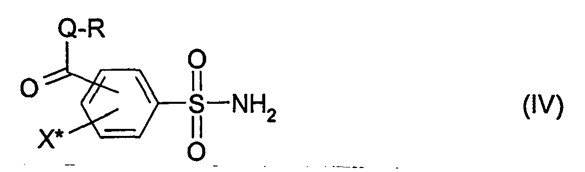 worin R, Q und X* wie in Formel (III) definiert sind,
      und die Verbindung (IV) mit oder ohne Zwischenisolierung mit Phosgen zum Phenylsulfonylisocyanat der Formel (V) umsetzt, 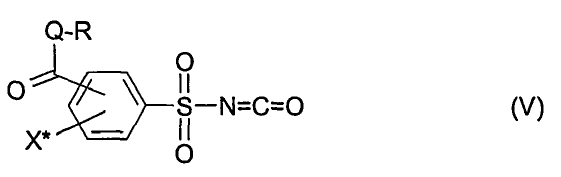 worin R, Q und X* wie in Formel (III) definiert sind,
      oder
   (b2) die erhaltene Verbindung (III) mit einem Cyanat, beispielsweise einem Alkalimetallcyanat, zu dem Isocyanat der Formel (V) oder einem solvatisierten (stabilisierten) Derivat davon umsetzt,
   und
(c) mit oder vorzugsweise ohne Zwischenisolierung das Isocyanat der Formel (V) oder dessen stabilisiertes Derivat mit einem heterocyclischen Amin der Formel (VI) 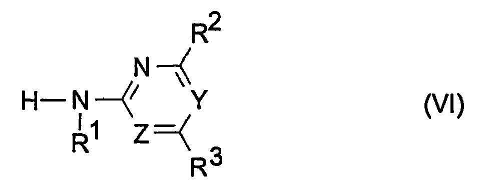 worin R¹, R², R³, Y und Z wie in Formel (I) definiert sind,
   zum Sulfonylharnstoff der Formel (I) oder einem Salz davon umsetzt.

Einige Verbindungen der Formel (II) (bestimmte Dihalogenide) sind neu und ebenfalls Gegenstand der Erfindung. Die Herstellung der Verbindungen der Formel (II) (Dihalogenide) sind ebenfalls Gegenstand der Erfindung (siehe weiter unten).

Weiterhin Gegenstand der Erfindung ist teilweise auch das Verfahren gemäß Teilschritt (a) (selektive Veresterung) unter Verwendung der Verbindungen (II). Einige selektive Veresterungen von Verbindungen (II) sind bereits bekannt; siehe beispielsweise die Umsetzung von 2,3,5,6-Tetrafluor-4-fluorsulfonyl-benzoylfluorid mit Methanol aus J. Fluorine Chem. 59, 1 1992, S. 15-32.
Weiterhin Gegenstand der Erfindung ist auch das Verfahren gemäß Teilschritt (b2) (Umsetzung unter Verwendung von Cyanat), vorzugsweise in Kombination mit dem Teilschritt (c).
In den Definitionen der Formeln (I) bis (VI) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein.

Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl, Alkenyl- und Alkinylreste haben die Bedeutung der den Alkylresten entsprechenden möglichen ungesättigten Reste, wobei mindestens eine Doppelbindung bzw. Dreifachbindung, vorzugsweise eine Doppelbindung bzw. Dreifachbindung enthalten ist. Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem mit vorzugsweise 3-8 C-Atomen, vorzugsweise 3 bis 6 C-Atomen, z.B. Cyclopropyl, Cyclobutyl, Cyclopentyl oder Cyclohexyl.

Halogen bedeutet beispielsweise Fluor, Chlor, Brom oder Iod. In Restedefinitionen wird mit "Halogen" ein Halogenrest, d. h. ein Halogenatom bezeichnet. Haloalkyl, -alkenyl und -alkinyl bedeuten durch Halogen, vorzugsweise durch Fluor, Chlor und/oder Brom, insbesondere durch Fluor oder Chlor, teilweise oder vollständig (durch gleiche oder verschiedene Halogenatome) substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein carbozyklisches aromatisches System, beispielsweise ein mono-, bi- oder polycyclisches aromatisches System, beispielsweise Phenyl, Naphthyl, Tetrahydronaphthyl, Indenyl, Indanyl, Pentalenyl, Fluorenyl und Ähnliches, vorzugsweise Phenyl.

Ein Kohlenwasserstoffrest enthält ausschließlich C-Atome und H-Atome und kann geradkettig, verzweigt oder cyclisch, gesättigt, ungesättigt oder aromatisch sein oder kann eine Kombination gleicher oder verschiedener der weiter oben genannten Kohlenwasserstoffreste enthalten. Beispielsweise werden von "Kohlenwasserstoffrest" die Reste Alkyl, Alkenyl, Alkinyl, Cycloalkyl, Cycloalkenyl, Cycloalkyl-alkyl, Cycloalkenyl-alkyl, Aryl wie Phenyl oder Naphthyl, Benzyl, Phenethyl etc. umfasst. Ein Kohlenwasserstoffrest enthält vorzugsweise 1 bis 30 C-Atome, insbesondere 1 bis 24 C-Atome, sofern nichts Anderes definiert ist.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste der Gruppe bzw. der generisch definierten Gruppe ein.

Substituierte Reste, wie ein substituierter Kohlenwasserstoffrest, z. B. substituierter Alkyl-, Alkenyl-, Alkinyl-, Aryl-, Phenyl-, Benzyl-rest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Alkylsulfinyl und Alkylsulfonyl und, im Falle cyclischer Reste, auch Alkyl, Haloalkyl, Alkylthio-alkyl, Alkoxy-alkyl, gegebenfalls substituiertes Mono- und Dialkylaminoalkyl und Hydroxy-alkyl bedeuten.
Bevorzugt sind Substituenten aus der Gruppe Halogen, Alkoxy, Alkylthio, Hydroxy, Amino, Nitro, Cyano, Mono- und Dialkylamino, im Falle cyclischer Reste, auch Alkyl und Haloalkyl;
im Begriff "substituierte Reste" wie substituierte Kohlenwasserstoffreste wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.
Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.
Bei den genannten Substituenten ist jeweils die Anzahl der C-Atome bevorzugt, wie sie weiter oben für Reste mit Kohlenwasserstoffanteilen als bevorzugt genannt sind.

Die Verbindungen der Formel (II) sind teilweise bekannt. So beschreibt US-A-4,110,373 die Reaktion von gegebenenfalls substituierten Benzotrichloriden mit Oleum. Unter anderem wird darin die Herstellung von 4-Chlor-3-chlorsulfonyl-benzoylchlorid und 3-Chlor-5-chlorsulfonyl-benzoylchlorid erwähnt.

Weiterhin ist aus NL-A-7603612 die Herstellung von 2-Chlorsulfonyl-benzoylchlorid aus 2-Sulfo-benzoesäure durch Reaktion mit Phosgen als Halogenierungsmittel in polaren aprotischen Lösungsmitteln wie DMF bekannt. Als Nebenprodukt tritt dabei in erheblichen Mengen Dichlortolylsulton (3,3-Dichlor-1,1-dioxo-benzo-1-thia-2-oxolane) auf. Das Verfahren wird allgemein auch für am Benzolring zusätzlich halogenierte oder nitrierte Derivate von Sulfo-benzoesäuren beschrieben.

Die Verbindungen (I) und (III) bis (VI) sind im Prinzip und teilweise spezifisch in den Druckschriften EP-A-007687, WO-A-92/13845 und WO-A-01/23368 und dort zitierter Literatur beschrieben. Insbesondere der Inhalt der WO-A-92/13845 und WO-A-01/23368 soll im übrigen hinsichtlich der Verfahrensvorschriften und der dort genannten bevorzugten Verbindungen der Formel (I) und deren Vorprodukten Bestandteil der vorliegenden Beschreibung und Erfindung sein.

Besonders bevorzugt sind für das Herstellungsverfahren Verbindungen der Formel (II), worin Q = Sauerstoffatom, X* = Wasserstoff oder Halogen, vorzugsweise Iod, R = (C₁-C₄)-Alkyl, (C₂-C₄)-Alkenyl, (C₂-C₄)-Alkinyl, (C₁-C₄)-Haloalkyl, oder (C₁-C₄)-Alkoxy(C₁-C₄)-alkyl, vorzugsweise Methyl oder Ethyl, insbesondere Methyl bedeutet. Vorzugsweise bedeuten Hal¹ = ein Chloratom und Hal² = ein Chloratom.
Ebenfalls bevorzugt sind Herstellungsverfahren mit Verbindungen der Formel (II), worin die Carbonsäurehalogenidgruppe in ortho-Stellung zur Sulfohalogenidgruppe steht. Weiter bevorzugt sind Herstellungsverfahren mit Verbindungen der Formel (II), worin die Carbonsäurehalogenidgruppe in ortho-Stellung zur Sulfohalogenidgruppe steht und X* = ein Halogenatom, vorzugsweise Iod, in para-Stellung zur Carbonsäurehalogenidgruppe steht.
Bevorzugt sind daher auch Verfahren, bei denen in Stufe a) Verbindungen der Formel (IIa) als Verbindungen der Formel (II) eingesetzt werden: 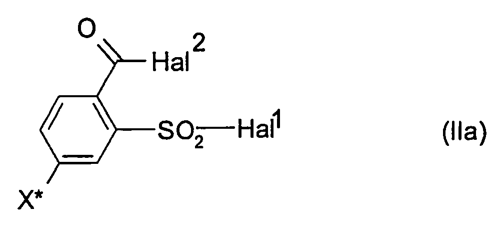 worin Hal¹ ein Halogenatom, vorzugsweise Chlor- oder Bromatom, insbesondere Chloratom bedeutet, Hal² ein Halogenatom, vorzugsweise ein Chlor- oder Bromatom, insbesondere Chloratom bedeutet und X* wie in Formel (I) definiert ist, vorzugsweise Halogen, insbesondere Iod bedeutet.
Generell sind Verfahren mit Zwischenprodukten und Verbindungen der Formel (I) bevorzugt, welche das Substitutionsmuster am Phenylring entsprechend Verbindungen (IIa) aufweisen.

Besonders bevorzugt sind auch erfindungsgemäße Verfahren, welche eine Kombination bevorzugt genannter Merkmale aufweisen.

Die erfindungsgemäße Reaktion der Verbindung der Formel (II) mit einer Verbindung der Formel R-Q-H oder einem Salz davon zur Verbindung der Formel (III) stellt eine selektive Reaktion des Dihalogenids mit dem Nucleophil R-Q-H dar.

Die Umsetzung wird mit einem Alkohol oder Thioalkohol (Q = Sauerstoffatom bzw. Schwefelatom) und/oder einem Salz davon durchgeführt, wobei das Salz beispielsweise direkt eingesetzt oder in der Reaktionsmischung aus dem Alkohol in Gegenwart einer anderen Base erzeugt werden kann. Die Reaktionsbedingungen werden zweckmäßig so gewählt, dass Nebenreaktionen an der SulfohalogenidGruppe möglichst vermieden werden. Als Nebenreaktionen kommen beispielsweise die Veresterung des Sulfohalogenids zum Sulfonsäureester mit nachfolgender intermolekularer Umesterung unter Bildung einer Sulfonsäuregruppe und weitere Folgereaktionen in Frage.

Verhältnismäßig guten Ausbeuten am gewünschten Monoester (III) der substituierten Halogensulfonylbenzoesäure werden beispielweise erhalten, wenn man die Umsetzung in einem inerten organischen Lösungs- und/oder Verdünnungsmittel (im Folgenden kurz als "Lösungsmittel" bezeichnet) unter Temperaturkontrolle durchführt. Als inerte organische Lösungsmittel kommen beispielsweise relativ unpolare aprotische organische Lösungsmittel in Frage, wie
- aliphatische und aromatische Kohlenwasserstoffe, wie z. B. Mineralöle, Petrolether, n-Pentan, n-Hexan, Cyclopentan, Cyclohexan bzw. Toluol, Xylole, Mesitylen, Naphthalinderivate, ®Solvesso 200 (hochsiedendes Aromatengemisch);
- halogenierte aliphatische und aromatische Kohlenwasserstoffe, wie Methylenchlorid, Dichlorethan bzw. Chlorbenzol, Chlortoluol oder Dichlorbenzol oder
- Gemische der genannten Lösungsmittel.

In größeren Ansätzen werden in der Regel die höhersiedenden organischen Lösungsmittel wie Toluol, Xylol, Mesitylen, Chlorbenzol, Chlortoluol oder Dichlorbenzol oder deren Gemische eingesetzt.

Als Alkohole oder Thioalkohole werden die dem Rest R-Q in Formel (I) entsprechenden Verbindungen R-Q-H eingesetzt. Bevorzugt sind dabei die (C₁-C₄)-Alkanole, wie Methanol, Ethanol, n-Propanol, i-Propanol, n-Butanol, i-Butanol, insbesondere Methanol und Ethanol. In der Regel werden 1 Moläquivalent oder ein Überschuss, beispielsweise 1 bis 20 Moläquivalente, vorzugsweise 1 bis 8 Moläquivalente, insbesondere 1 bis 6 Moläquivalente der Verbindung R-Q-H pro Mol Verbindung der Formel (II) (Dihalogenid, z. B. Dichlorid) eingesetzt. Im Falle Methanol werden vorzugsweise 1 bis 8 Moläquivalente, insbesondere 1 bis 6 Moläquivalente, ganz besonders 3 bis 6 Moläquivalente eingesetzt.

Als Salze der Alkohole oder Thioalkohole kommen beispielsweise die Alkalimetallsalze, vorzugsweise Natrium- oder Kaliumsalze, oder Erdalkalimetallsalze in Frage. In der Regel werden 1 Moläquivalent oder ein Überschuss, beispielsweise 1 bis 10 Moläquivalente, vorzugsweise 1 bis 3 Moläquivalente, insbesondere 1 bis 2 Moläquivalente Salz der Verbindung R-Q-H pro Mol Verbindung der Formel (II) (Dihalogenid) eingesetzt. Im Falle eines Salzes von Methanol, beispielsweise Natriummethylat, werden vorzugsweise 1 bis 10 Moläquivalente, insbesondere 1 bis 3 Moläquivalente, ganz besonders 1 bis 2 Moläquivalente Methylat eingesetzt.

Die Reaktionstemperatur für die Veresterung kann je nach Komponenten und deren Anteilen in der Reaktionsmischung zweckmäßig mit Hilfe von Vorversuchen optimiert werden und liegt meist im Bereich von -20 °C bis 100 °C. Für den Fall, dass ein Alkohol oder Thioalkohol, vorzugsweise ein (C₁-C₄)-Alkanol, insbesondere Methanol zur Veresterung eingesetzt wird, liegt eine geeignete Reaktionstemperatur In der Regel im Bereich von -10 °C bis 70 °C, vorzugsweise 20 bis 40°C. Im Falle des Einsatzes von Salzen der Verbindungen R-Q-H liegt die optimale Reaktionstemperatur vergleichsweise meist niedriger. In der Regel ist eine geeignete Reaktionstemperatur bei der Veresterung mit einem Salz, vorzugsweise ein Alkalimetallsalz oder Erdalkalimetallsalz eines (C₁-C₄)-Alkanols oder auch Thioalkohols, insbesondere Natriummethylat oder Kaliummethylat, im Bereich von -20 °C bis 50 °C, vorzugsweise -10 bis 35 °C, insbesondere 0 bis 25 °C.

Zur Aufarbeitung des Reaktionsansatzes kann gemäß üblichen Methoden verfahren werden. Nach vollständiger Reaktion mit einem Überschuss an Alkohol kann überschüssiger Alkohol beispielsweise im Vakuum abdestilliert werden und dann die Reaktionsmischung zum Entfernen entstandenes Salzes auf Wasser gegeben und anschließend das Produkt mit einem organischen Lösungsmittel extrahiert werden. Alternativ kann der Ansatz direkt in Wasser gegeben und mit einem Lösungsmittel extrahiert werden.

Die weitere Umsetzung des erhaltenen Halogensulfonylbenzoesäureesters (III) (z. B.

Chlorsulfonylbenzoesäureesters) kann gemäß oder analog den bekannten Verfahrensweisen, wie sie beispielsweise in WO-A-92/13845 und WO 01/23368 und dort zitierter Literatur beschrieben sind, durchgeführt werden. Die bekannte Verfahrensweise beinhaltet die Ammonolyse der Verbindung der Formel (III) zum Sulfonamid der Formel (IV), Phosgenierung von Verbindung (IV) zum Phenylsulfonylisocyanat der Formel (V) und anschließende Additionsreaktion (Kupplung) mit einem heterocyclischen Amin der Formel (VI) zum Sulfonylharnstoff der Formel (I) oder dessen Salz. Auf die Verfahrensweisen für die Durchführung der Ammonolyse, Phosgenierung und Kupplung wird diesbezüglich spezifisch auf die WO 01/23368 Bezug genommen.

Alternativ zu der vorstehenden Verfahrensweise kann die Verbindung der Formel (III) mit einem Cyanat, beispielsweise einem Cyanatmetallsalz, insbesondere einem Alkalimetallcyanat, zu dem Isocyanat der Formel (V) oder einem solvatisierten (stabilisierten) Derivat davon umgesetzt werden.

Als Cyanate eignen sich Cyanate mit Kationen aus der Gruppe der Metallkationen oder der organischen Kationen wie sterisch gehinderte organische Ammoniumionen. Bevorzugt sind beispielsweise Alkalimetallcyanate, vorzugsweise Natriumcyanat und Kaliumcyanat, oder auch Erdalkalimetallcyanate. Das verwendete Cyanat oder Cyanatgemisch wird zweckmäßig in einer Menge eingesetzt, welche für die Umsetzung zur Verbindung (V) ausreichend ist. In der Regel reicht dafür eine äquimolare Menge an Cyanat oder ein geringer Überschuss, vorzugsweise 1 bis 2 Moläquivalente, insbesondere 1 bis 1,5 Moläquivalente an Cyanat bezogen auf Verbindung (III) aus.

Die Umsetzung mit dem Cyanat wird in der Regel in einem aprotischen polaren Lösungsmittel durchgeführt. Geeignete Lösungs- und/oder Verdünnungsmittel sind aprotische organische Lösungsmittel, die unter den Reaktionsbedingungen inert sind, beispielsweise wie
- Ether, wie Diethylether, Di-n-propylether, Diisopropylether, Methyl-tert-butylether, Tetrahydrofuran (THF), Dioxan, Alkylenglykolmonoalkylether und -dialkylether wie z. B. Propylenglykolmonomethylether, Propylenglykolmonoethylether, Ethylenglykolmonomethylether oder -monoethylether, Dimethoxyethan, Diglyme, Triglyme und Tetraglyme; Amide, wie Dimethylformamid (DMF), Dimethylacetamid und N-Methylpyrrolidon;
- Ketone, wie Aceton, Cyclohexanon, Methylisobutylketon (MIBK);
- Nitrile, wie Acetonitril, Propionitril, Butyronitril und Benzonitril;
- Sulfoxide und Sulfone, wie Dimethylsulfoxid (DMSO) und Sulfolan
sowie Gemische aus zwei oder mehreren der vorstehend genannten Lösungs- oder Verdünnungsmitteln.

Verfahrenstechnisch von besonderem Interesse sind in der Regel solche Lösungsmittel, welche gut durch Destillation aus dem Produkt entfernt werden können.
Die Reaktionszeit kann je nach Korngröße des Cyanats, sofern es in der Reaktionsmischung als Festkörper vorliegt, variieren.

Bevorzugt sind aprotische Solventien aus der Gruppe der Ether, z. B. Diisopropylether und Methyl-tert.-butylether, Ketone, z.B. Methylisobutylketon (MIBK), und der Nitrile wie Acetonitril.

Es ist meist vorteilhaft sein, die Reaktion zum Isocyanat der Formel (V) unter Zusatz von N-Heteroaromaten (Stickstoffheterocyclen) als Katalysator oder Stabilisator in der Reaktionsmischung, beispielsweise durch Pyridin und Pyridinderivate der Formel (VII), 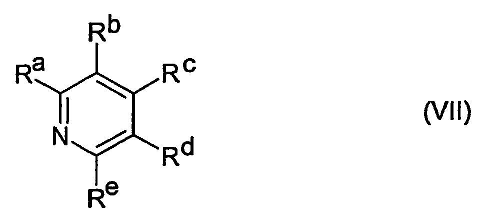 worin
R^{a}, R^{b}, R^{c}, R^{d} und R^{e} jeweils unabhängig voneinander Wasserstoff, (C₁-C₆)Alkyl, (C₂-C₆)Alkenyl, (C₂-C₆)Alkinyl oder (C₁-C₆)Alkoxy bedeuten oder zwei benachbarte Reste gemeinsam mit den sie verbindenenden C-Atomen des ersten Ringes einen ankondensierten carbocyclischen Ring mit 4 bis 8 C-Atomen oder einen heterocyclischen Ring mit 4 bis 8 Ringatomen und 1, 2 oder 3 Heteroringatomen aus der Gruppe N, O und S bilden, durchzuführen.
Vorzugsweise bedeuten R^{a}, R^{b}, R^{c}, R^{d} und R^{e} jeweils unabhängig voneinander Wasserstoff oder ein, zwei oder drei der Reste bedeuten (C₁-C₄)Alkyl oder (C₁-C₄)Alkoxy, insbesondere Methyl oder Ethyl, und die übrigen bedeuten jeweils Wasserstoff.
Beispiele für geeignete N-Heteroaromaten sind Pyridin oder substituierte Pyridine wie Alkylpyridine, beispielsweise Picoline (z. B. 2-Methylpyridin, 3-Methylpyridin oder 4-Methylpyridin) oder Lutidine (z. B. 2,4-Dimethylpyridin, 2,6-Dimethylpyridin, 2,3-Dimethylpyridin, 2,5-Dimethylpyridin, 3,4-Dimethylpyridin oder 3,5-Dimethylpyridin), oder deren Gemischen. Die Menge an N-Heteroaromaten kann in einem breiten Bereich variieren. Zweckmäßig werden 0,8 bis 2 Moläquivalente, vorzugsweise 0,9 bis 1,5 Moläquivalente, insbesondere 1 bis,1,3 Moläquivalente des N-Heteroaromaten pro Mol Halogensulfonylbenzoesäureester (III), vorzugsweise Chlorsulfonylbenzoesäureester, bezogen auf ein Mol des zu entstehenden Isocyanats (V) eingesetzt.

Im Falle von Y = CH (Pyrimidin-2-yl) kann die Zugabe von katalytischen Mengen an Pyridin oder eines Pyridinderivats bei der Herstellung des Isocyanats in Gegenwart eines Cyanats ausreichend sein.

Bei der Zugabe eines Stabilisators aus der Gruppe der N-Heteroaromaten liegt das Produkt ganz oder teilweise nicht in der Form des Isocyanats vor, sondern in Form eines in Lösung stabilen Zwischenprodukts, das im im Falle eines Einsatzes von bevorzugt genannten Pyridin(derivaten) der Formel (VII) eine Verbindung der Formel (VIII), 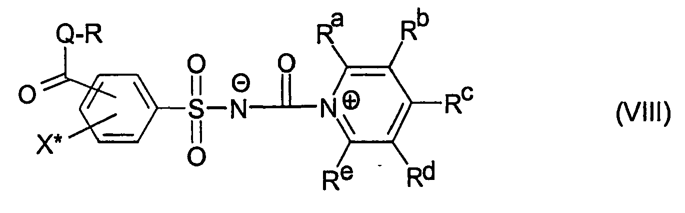 worin R, R^{a}, R^{b}, R^{c}, R^{d}, R^{e}, Q und X* wie in Formel (III) bzw. Formel (VII) definiert sind, bedeutet.
Im Falle von Pyridin als Stabilisator entsteht entsprechend das Zwischenprodukt der Formel (VIIIa), 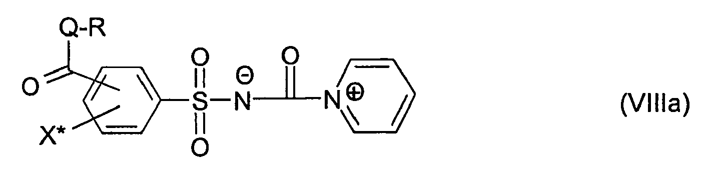 worin
R, Q und X* wie in Formel (III) definiert sind.

Die Zwischenprodukte der Formel (VIII) bzw. (VIIIa) sind neu und deshalb auch ein Gegenstand der Erfindung. Besonders bevorzugt sind die Zwischenprodukte der Formel (VIIIb) 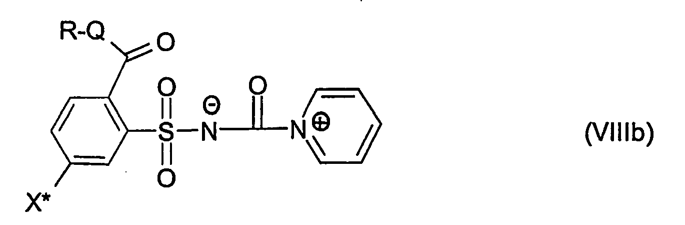 worin
R, Q und X* wie in Formel (III) definiert sind.

Die Verbindungen der Formeln (VIII), (VIIIa) und (VIIIb) lassen sich beispielsweise spektroskopisch nachweisen. Sie zeichnen sich durch eine charakteristische Verschiebung der Bande der Carbonyl-Schwingung im Infrarot-Spektrum im Vergleich zur Carbonyl-Bande der entsprechenden Isocyanate der Formel (V) aus.

Die Reaktionstemperatur für die Umsetzung von Verbindung (III) mit dem Cyanat kann in weiten Grenzen variiert und in Vorversuchen optimiert werden. Sie liegt vorzugsweise bei moderaten Werten im Bereich von -30 °C bis 70 °C, insbesondere von -10 °C bis 30 °C.

Das Isocyanat (V) kann anschließend analog zum Isocyanat aus Variante (b1) mit einem heterocyclischen Amin (VI) zum Sulfonylharnstoff (I) umgesetzt werden. Zweckmäßig wird für den Fall eines Zusatzes von Katalysator oder Stabilisator zunächst mit einer Säure, vorzugsweise wasserfreien Säure, beispielsweise Chlorwasserstoff oder einer organischen Säure, neutralisiert.

In einer Variante des Verfahrens wird die Reaktionsmischung aus der Herstellung des Isocyanats nach dem Cyanat-Verfahren direkt für die Kupplung des Isocyanats oder stabilisierten Isocyanats zum Sulfonylharnstoff der Formel (I) eingesetzt. Dazu wird gegebenenfalls enthaltender N-Heteroaromat durch Zugabe einer Säure neutralisiert (wie oben erwähnt) und die Reaktionsmischung mit dem heterocyclischen Amin (VI) versetzt. Alternativ kann das heterocyclische Amin (VI) zugegeben werden und dann erst die Säure zur Neutralisation des Katalysators/Stabilisators zugegeben werden.

Die Umsetzung der Verbindungen (V) und (VI) werden in der Regel in einem organischen Lösungsmittel durchgeführt. Als Lösungsmittel können dabei polare oder relativ unpolare aprotische Lösungsmittel eingesetzt werden. Vorzugsweise werden bei der Umsetzung dieselben Lösungsmittel verwendet wie bei der Herstellung des Isocyanats (V).

Die Reaktion wird bevorzugt bei einer Temperatur im Bereich von 0 °C bis zum Siedepunkt des Lösungmittels, vorzugsweise 0 °C bis 100°C, insbesondere 20 bis 80 °C, ganz besonders 20 bis 40 °C durchgeführt.

Bezogen auf ein Mol Sulfonylisocyanat der Formel (V) werden vorzugsweise 1 bis 1,2 Mol, insbesondere 1 bis 1,1 Mol, ganz besonders 1 bis 1,05 Mol Amin der Formel (VI) eingesetzt.

Die Aufarbeitung des Reaktionsgemischs aus der Kupplung kann nach üblichen Methoden erfolgen, wobei die Sulfonylharnstoffe der Formel (I) beispielsweise als Nichtsalze oder - nach Umsetzung mit Basen oder gegebenenfalls auch Säuren - als Salze isoliert werden können.

Das erfindungsgemäße Verfahren ermöglicht die Herstellung von Sulfonylharnstoffen (I) oder deren Salzen auf einfachem Wege ausgehend vom Dichlorid der Formel (II) in relativ guten bis excellenten Ausbeuten in drei bzw. vier Verfahrensstufen. In der erfindungsgemäßen Variante mit drei Verfahrensschritten wird eine Phosgenierung vermieden.

Die Verbindungen der Formel (II) können hergestellt werden, in dem man eine Verbindung der Formel (IX) oder ein Salz davon, 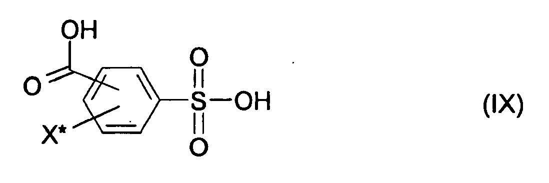 worin X* wie in Formel (II) definiert ist,
mit einem oder mehreren Halogenierungsmitteln aus der Gruppe der Säurehalogenide des Schwefels oder Phosphors in einer oder mehreren Reaktionsschritten in Gegenwart eines Katalysators aus der Gruppe der sterisch gehinderten Aminbasen zur Verbindung der Formel (II), vorzugsweise dem Dichlorid, umsetzt.

Die Verbindungen der Formel (IX) sind beispielsweise aus WO 95/26952 bekannt oder können analog den bekannten Verfahren hergestellt werden.

Die Verbindungen der Formel (II) sind teilweise bekannt. So beschreibt US-A-4,110,373 die Reaktion von gegebenenfalls substituierten Benzotrichloriden mit Oleum. Unter anderem wird darin die Herstellung von 4-Chlor-3-chlorsulfonyl-benzoylchlorid und 3-Chlor-5-chlorsulfonyl-benzoylchlorid erwähnt.
Weiterhin ist aus NL-A-7603612 (= DE-A-2616612) die Herstellung von 2-Chlorsulfonyl-benzoylchlorid aus 2-Sulfo-benzoesäure durch Reaktion mit Phosgen als Halogenierungsmittel in polaren aprotischen Lösungsmitteln wie DMF bekannt. Als Nebenprodukt tritt dabei in erheblichen Mengen Dichlortolylsulton (3,3-Dichlor-1,1-dioxo-benzo-1-thia-2-oxolane) auf. Das Verfahren wird allgemein auch für am Benzolring zusätzlich halogenierte oder nitrierte Derivate von Sulfo-benzoesäuren beschrieben. Aus American Chem. Journal 30 (1893), 485-517 ist die Dichlorierung von p-Brom-ortho-chlorsulfonylbenzoesäurechlorid aus p-Brom-orthosulfobenzoesäure mit Phosphorpentachlorid bekannt. Weiterhin ist in American Chemical Journal 50 (1913), 193-204 die Dichlorierung von Nitro-orthosulfobenzoesäure mit Phosphorpentachlorid zu Nitro-ortho-chlorsulfonylbenzoesäurechlorid beschrieben.
Die Verbindungen der Formel (II) bzw. Formel (IIa), worin die Halosulfonylgruppe in Orthostellung zur Carbonsäurehalogenidgruppe steht und die in Parastellung zur Carbonsäurehalogenidgruppe zusätzlich iodiert sind, sind ebenfalls neu und Gegenstand der Erfindung (Verbindungen (IIa)).

Wegen der bekannten Halogenierungsmethode mit Phosgen, die zu Nebenreaktionen führt, bestand die Aufgabe, ein alternatives Verfahren bereitzustellen, welches die Verbindungen (II) oder (IIa) herzustellen erlaubt.

Als Halogenierungsmittel kommen anorganische Säurehalogenide des Schwefels und Phosphors in Frage, beispielsweise Thionylhalogenide, wie Thionylfluorid oder Thionylchlorid, oder Sulfurylhalogenide, wie Sulfurylchlorid, oder Phosporhalogenide wie Phosphortrichlorid, Phosphorylchlorid, Phosphorpentachlorid, Phosphortribromid. Vorzugsweise ist die Herstellung von Dichloriden (Formel (II) oder (IIa), worin Hal¹ = Cl und Hal² = Cl), vorzugsweise mit Thionylchlorid, Phosphortrichlorid, Phosphorylchlorid oder Phosphorpentachlorid, insbesondere mit Thionylchlorid.

Das Halogenierungsmittel wird in der Regel in einer Menge von einem Reaktionsäquivalent pro reaktiver Gruppe oder im Überschuss eingesetzt. Dabei sind die üblichen bekannten Reaktionsäquivalente zu berücksichtigen, z. B. beim Chlorierungsmittel Thionylchlorid 1 Reaktionsäquivalent, bei PCI₃ und POCI₃ drei Reaktionsäquivalente, bei PCI₅ je nach Reaktionsbedingungen ein oder vier Reaktionsäquivalente.

Pro Mol Verbindung der Formel (IX) werden nach der Stöchiometrie mindestens 2 Äquivalente an Halogenierungsmittel benötigt. In der Regel sind je nach Halogenierungsmittel 2 bis 10 Äquivalente an Halogenierungsmittel pro Mol Disäure ausreichend. Im Falle von Thionylchlorid werden vorzugsweise 4 bis 8 Mol pro Mol Verbindung der Formel (IX) eingesetzt. Überschüssiges Halogenierungsmittel und gegebenenfalls entstandener Halogenwasserstoff werden vorzugsweise kontinuierlich während der oder nach der Reaktion von dem Reaktionsprodukt abgetrennt oder chemisch gebunden. Im Falle von Thionylchlorid ist es meist möglich, einen Überschuss vom Produkt abzudestillieren.

Die Halogenierung der Verbindung (IX) wird in der Regel in Gegenwart eines inerten (relativ) unpolaren organischen Lösungsmittels durchgeführt, kann aber in Einzelfällen auch in Substanz durchgeführt werden. Als Lösungsmittel kommen zahlreiche inerte Lösungsmittel, vorzugsweise weitgehend unpolare Lösungsmittel in Frage, welche unter den Halogenierungsbedingungen nicht oder weitgehend nicht mit dem Halogenierungsmittel oder dem Reaktionsprodukt reagieren. Beispiele für geeignete Lösungsmittel sind:
- aliphatische und aromatische Kohlenwasserstoffe, wie z. B. Mineralöle, Petrolether, n-Pentan, n-Hexan, Cyclopentan, Cyclohexan bzw. Toluol, Xylole, Mesitylen, Naphthalinderivate, ®Solvesso 200 (hochsiedendes Aromatengemisch);
- halogenierte aliphatische und aromatische Kohlenwasserstoffe, wie Methylenchlorid, Dichlorethan bzw. Chlorbenzol, Chlortoluol oder Dichlorbenzol
sowie Gemische aus zwei oder mehreren der vorstehend genannten Lösungs- oder Verdünnungsmitteln. Vorteilhaft eignen sich solche inerten Lösungsmittel, welche in der Vorstufe oder der nachfolgenden Stufe des Gesamtverfahrens ebenfalls verwendet werden können. Beispielsweise eignen sich dafür gegebenenfalls halogenierte aromatische Kohlenwasserstoffe, vorzugsweise hochsiedende organische Lösungsmittel wie Xylol, Toluol, Mesitylen, Chlorbenzol oder Dichlorbenzol oder deren Gemische.

Die Halogenierungsreaktion wird durch Zugabe von polaren wenig nucleophilen basischen Verbindungen katalysiert solche sind sterisch gehinderte Aminbasen, z. B. trisubstituierten Aminen oder Stickstoffheterocyclen. Prinzipiell geeignet sind beispielsweise Triethylamin, Pyridin, Alkylpyridine (z. B. Picoline, Lutidine), DBU (1,8-Diazabicyclo[5.4.0]undec-7-en), DABCO (1,4-Diazabicyclo[2.2.2]octane) oder Amide, wie die Dialkylformamide DMF (Dimethylformamid), Diethylformamid, Di-n-propylformamid, Di-isopropylformamid, Di-n-butylformamid oder Di-n-Pentylformamid, oder Dimethylacetamid. Aus praktischen Gründen wie Erleichterung der Aufarbeitung, Reinigung oder Effizienz, sollte der Anteil an Katalysator möglichst gering gewählt werden. Oft sind Mengen von 0,01 bis 2 Moläquivalente, vorzugsweise 0,02 bis 1 Moläquivalente Katalysator bezogen auf Verbindung der Formel (IX) für eine Beschleunigung der Reaktion ausreichend. Die geeigneten Reaktionstemperaturen können in Abhängigkeit von Halogenierungsmittel und Katalysator variieren und in Vorversuchen einfach bestimmt werden. Sie liegen in der Regel im Bereich von -10 °C bis zum Siedepunkt des jeweiligen Lösungsmittels, vorzugsweise 20 °C bis 150 °C, insbesondere 40 °C bis 120 °C, wobei die Untergrenze der Reaktionstemperatur durch einen feststellbaren Umsatz festgelegt ist. Gegenstand der Erfindung ist auch die Halogenierungsreaktion in Gegenwart dieser Katalysatoren.

Im Prinzip kann die Reaktion durchgeführt werden, indem das Edukt der Formel (IX), meist in Substanz oder gelöst oder suspendiert im Lösungsmittel, mit dem Katalysator bei einer erhöhten Temperatur (oberhalb der Temperatur, bei der eine Reaktion einsetzen würde) vorgelegt wird und dann das Halogenierungsreagenz zudosiert wird. Trotz der erhöhten Temperatur kann es vorkommen, dass die Reaktion verzögert einsetzt, die Reaktion relativ langsam verläuft und/oder unvollständig abläuft.

Alternativ dazu kann man das Halogenierungsreagenz, vorzugsweise Chlorierungsreagenz mit dem Katalysator bei einer erhöhten Temperatur, vorzugsweise 60 bis 80 °C vorlegt werden und dann das Edukt zudosiert werden, entweder als Feststoff in Substanz oder in Suspension im Lösungsmittel. Damit kann ein großer Überschuss von Edukt im Reaktor vermieden werden.

Nach vollständiger Reaktion wird der Überschuß an Halogenierungsreagenz vorzugsweise abdestilliert, was gegebenenfalls unter reduziertem Druck erfolgen kann. Wegen der Reaktionsfähigkeit des Dihalogenids der Formel (II) wird dieses nach der Herstellung vorzugsweise ohne Zwischenisolierung direkt weiterverarbeitet, d. h. beispielsweise für die oben beschriebene Monoveresterung zur Verbindung der Formel (III) oder deren Salze.

In den folgenden Beispielen beziehen sich Mengenangaben auf das Gewicht, sofern nicht speziell andere Definitionen angegeben sind.

### Beispiel 1 Herstellung eines Dihalogenids

### Herstellung von 2-Chlorsulfonyl-4-iod-benzoylchlorid durch Zugabe von festem Kalium 3-lod-6-carboxybenzolsulfonat

650 g (5,3 mol) Thionylchlorid (technisch, >97%ig) werden mit Stickstoff überlagert, auf 70°C erhitzt und es werden langsam 8 g (0,11 mol) Dimethylformamid zugegeben. Nach beendeter Zugabe läßt man 15 Minuten bei der angegebenen Temperatur rühren, bevor über eine Feststoffschnecke 242 g (0,661 mol) 3-lod-6-carboxybenzolsulfonsäure-monokaliumsalz so schnell zugegeben wird, dass ein gut kontrollierbarer Abgasstrom entsteht. Nach beendeter Zugabe wird die Innentemperatur langsam bis auf 85 °C erhöht und für 2 Stunden bei dieser Temperatur gehalten. Überschüssiges Thionylchlorid wird dann bei einer Temperatur von 90 - 100 °C abdestilliert. Während der Destillation wird der Druck bis minimal 180 mbar reduziert. Man erhält eine Schmelze des Produktes mit suspendiertem feinkristallinen Kaliumchlorid.
Zum Entfernen von restlichem Thionylchlorid werden 100 ml Xylol zugegeben und im Vakuum abdestilliert. Die erhaltene Suspension kann nach Zugabe von Xylol ohne weitere Aufarbeitung in eine nachfolgende Veresterung eingesetzt werden. Alternativ kann aufgearbeitet werden, indem man die Mischung mit 400 ml Xylol versetzt und nach Abkühlen auf ca. 30 °C unter Schutzgas filtriert. Der Niederschlag wird zweimal mit Xylol gewaschen, und die vereinigten Filtrate werden im Vakuum vom Lösungsmittel befreit. Man erhält 238 g (0,652 mol = 98,6% d. Th.) eines schwach gelblichen Feststoffes (Reinheit > 99%, HPLC).

### Beispiel 2 Herstellung eines Dihalogenids

### Herstellung von 2-Chlorsulfonyl-4-iod-benzoylchlorid unter Zugabe einer xylolischen Suspension von Kalium 3-lod-6-carboxybenzolsulfonat

650 g (5,3 mol) Thionylchlorid (technisch, >97%ig) werden mit Stickstoff überlagert, auf 70°C erhitzt und es werden langsam 8 g (52 mmol) Di-n-butylformamid zugegeben. Nach beendeter Zugabe läßt man 15 Minuten bei der angegebenen Temperatur rühren. Die Temperatur wird auf 85 °C erhöht, bevor eine gut gerührte Suspension von fein gemahlenem 242 g (0,74 mol) 3-lod-6-carboxybenzolsulfonsäure-monokaliumsalz in 400 ml Xylol so schnell zugegeben wird, dass ein gut kontrollierbarer Abgasstrom entsteht. Nach beendeter Zugabe wird mit wenig Xylol nachgewaschen und für 2 Stunden bei dieser Temperatur gehalten. Überschüssiges Thionylchlorid wird dann über eine Fraktionierkolonne bei einer Temperatur von 90 - 100°C abdestilliert. Während der Destillation wird der Druck bis minimal 180 mbar reduziert. Es wird destilliert, bis am Kolonnenkopf der Siedepunkt von Xylol beim entspechenden Druck stabil erreicht ist. Gegebenenfalls wird abdestilliertes Xylol durch Zugabe von frischem Xylol ergänzt.
Die erhaltene Reaktionsmischung kann wie in Beispiel 1 aufgearbeitet werden oder direkt für eine nachfolgende Veresterung eingesetzt werden.

### Beispiel 3 Herstellung eines Dihalogenids

### Herstellung von 2-Chlorsulfonyl-4-iod-benzoylchlorid durch Umsetzung von 4-lod-2-sulfo-benzoesäure

20,9 g 4-lod-2-sulfo-benzoesäure werden in 80 ml Phosphoroxychlorid (POCl₃) suspendiert. Unter Rühren gibt man 26,7 g Phosphorpentachlorid (PCl₅) zu. Man erhitzt langsam auf eine Temperatur von 100-110 °C, wobei ab ca. 40 °C Innentemperatur heftige Gasentwicklung einsetzt. Nach 1 Stunde Reaktionszeit wird die Reaktionsmischung auf Raumtemperatur abgekühlt und das Lösungsmittel am Rotationsverdampfer abgezogen. Man erhält 28,3 g rohes 2-Chlorsulfonyl-4-iod-benzoylchlorid.

### Beispiel 4 Herstellung einer Verbindung (III)

### Herstellung von 2-Chlorsulfonyl-4-iod-benzoesäuremethylester

Eine wie in Beispiel 1 oder 2 erhaltene Reaktionsmischung wird auf 20- 25°C abgekühlt und es werden 140 ml (110,6 g) Methanol so zugetropft, dass die Innentemperatur 28 °C nicht übersteigt. Nach beendeter Zugabe wird bis zur vollständigen Umsetzung gerührt und nach Variante A oder B aufgearbeitet.

Variante A: Überschüssiges Methanol wird im Vakuum bei einer Temperatur von weniger als 30 °C gemeinsam mit etwas Xylol vollständig abdestilliert. Es wird mit Xylol auf ein Gesamtvolumen von 1320 ml verdünnt und in drei Portionen mit Wasser gewaschen. Die organische Phase wird abgetrennt und durch azeotrope Destillation im Vakuum getrocknet. Das Produkt kann für eine nachfolgende Reaktion in Form der xylolischen Lösung eingesetzt werden.
Alternativ wird das Lösungmittel im Vakuum abdestilliert und man erhält 237 g (= 99.5% telquel*) der Titelverbindung als erstarrte Schmelze mit einer Reinheit von 98%(HPLC), (97,4% d.Th.*).
* = bezogen auf eingesetztes 3-lod-6-carboxybenzolsulfonsäure-monokaliumsalz

Variante B: Es wird mit Xylol auf ein Gesamtvolumen von 1320 ml verdünnt und in drei Portionen mit Wasser gewaschen. Die organische Phase wird abgetrennt und durch azeotrope Destillation im Vakuum getrocknet. Das Produkt kann für eine nachfolgende Reaktion in Form der xylolischen Lösung eingesetzt werden. Alternativ wird das Lösungmittel im Vakuum abdestilliert und man erhält 235 g (= 98.6% telquel *) der Titelverbindung als erstarrte Schmelze mit einer Reinheit von 97%(HPLC), (95.6% d.Th.*).
* = bezogen auf eingesetztes 3-lod-6-carboxybenzolsulfonsäure-monokaliumsalz

### Beispiel 5: Herstellung einer Verbindung (III) aus einem Säuredichlorid

### Herstellung von 2-Chlorsulfonyl-4-iod-benzoesäureprop-2-inylester

28,3 g des rohen Säuredichlorids wie aus Beispiel 4 werden in 150 ml Chloroform gelöst und mit 7,7 ml Propargylalkohol versetzt. Man erhitzt 3 Stunden in der Siedehitze, kühlt auf Raumtemperature ab und gießt auf 200 ml Eiswasser. Man stellt die wässrige Phase mit NaHCO₃ neutral, trennt die Phasen und extrahiert die Wasserphase noch zweimal mit Dichlormethan. Die vereinigten organischen Phasen werden über Na₂SO₄ getrocknet, und das Lösungsmittel wird am Rotationsverdampfer abgezogen. Man erhält 23,5 g rohen 2-Chlorsulfonyl-4-iod-benzoesäureprop-2-inylester.

### Beispiel 6: Herstellung einer Verbindung (III) aus einer Halogen-sulfobenzoesäure

### Herstellung von 2-Chlorsulfonyl-4-iod-benzoesäureprop-2-inylester

98,4 g 4-lod-2-sulfo-benzoesäure werden in einer Lösung von 33,7 g Kaliumhydroxid (Plätzchen) in 150 ml Wasser und 500 ml Methanol gelöst. Das Lösungsmittel wird dann am Rotationsverdampfer abgezogen und der Rückstand am Hochvakuum getrocknet. Man erhält 133,9 g rohes Di-Kaliumsalz der Ausgangsverbindung. Zu 66 g dieses Salzes tropft man 150 ml Thionylchlorid zu und gibt noch 4,7 ml Dimethylformamid (DMF) hinzu. Man erhitzt bis zum Ende der Gasentwicklung in der Siedehitze und zieht dann das Lösungsmittel unter Feuchtigkeitsausschluß am Rotationsverdampfer ab. Dann tropft man unter Kühlung 150 ml Propargylalkohol zu und rührt 6 Stunden bei Raumtemperatur nach. Die Reaktionslösung wird eingedampft, mit 150 ml Wasser versetzt, abgesaugt und der Filterkuchen gut mit Wasser nachgewaschen. Der Filterrückstand wird in Dichlormethan suspendiert und filtriert und die organische Phase eingeengt. Sie enthält 35,2 g 2-Chlorsulfonyl-4-iod-benzoesäureprop-2-inylester vom Schmelzpunkt 69-73 °C. Zusätzlich enthält die Phase 14,3 g 4-lod-2-sulfo-benzoesäure als Hydrolyseprodukt.

### Beispiel 7: Herstellung von 4-lod-[3-(4-methoxy-6-methyl-1,3,5-triazin-2-yl)-ureidosulfonyl]-benzoesäuremethylester

106 g 2-Chlorsulfonyl-4-iod-benzoesäuremethylester (97%ig) werden in 500 g Acetonitril gelöst und mit 23 g technischem Natriumcyanat (95 %) versetzt. Die Mischung wird auf 6-10°C gekühlt und es werden 25 g Pyridin in 100 ml Acetonitril innerhalb von 2-4 Stunden zugegeben und bis zum kompletten Umsatz gerührt (Herstellung des stabilisierten Isocyanats).
Dann werden 43 g technisches 2-Amino-4-methoxy-6-methyl-1,3,5-triazin zugesetzt, und es wird auf 0 °C abgekühlt. Es werden dann 12 g trockenes ChlorwasserstoffGas unter die Oberfläche geleitet und nach beendeter Einleitung bei 40 °C bis zur vollständigen Umsetzung gerührt. Nach Abdestillieren des Acetonitrils unter reduziertem Druck wird der Niederschlag abfiltriert und mit Acetonitril nachgewaschen. Der Niederschlag wird mit einer Mischung aus Aceton und verdünnter Salzsäure aufgeschlemmt erneut filtriert, mit Wasser und Aceton gewaschen und im Vakuum getrocknet. Man erhält 114 g (77,3 % d. Th.) der Titelverbindung als weißes Pulver (Reinheit: mehr als 98%, HPLC).

## Patentansprüche

1. Verfahren zur Herstellung der Verbindung der Formel (I) oder deren Salze, 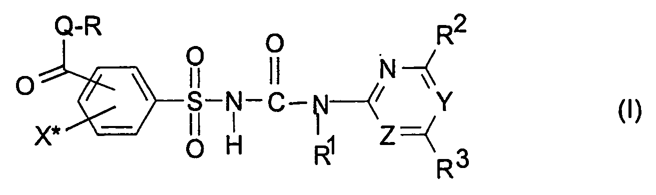 worin
Q Sauerstoff oder Schwefel,
X* Wasserstoff, Halogen, Cyano, Nitro, (C₁-C₃)-Alkyl oder Methoxy,
Y,Z unabhängig voneinander CH oder N, wobei Y und Z nicht gleichzeitig CH sind,
R Wasserstoff, (C₁-C₁₂)-Alkyl, (C₂-C₁₀)-Alkenyl, (C₂-C₁₀)-Alkinyl, (C₁-C₆)-Alkyl, das ein- bis vierfach durch Reste aus der Gruppe Halogen, (C₁-C₄)-Alkoxy, (C₁-C₄)-Alkylthio, CN, [(C₁-C₄)-Alkoxy]-carbonyl und (C₂-C₆)-Alkenyl substituiert ist, oder (C₃-C₈)-Cycloalkyl, das unsubstituiert oder durch Reste aus der Gruppe (C₁-C₄)-Alkyl, (C₁-C₄)-Alkoxy, (C₁-C₄)-Alkylthio und Halogen substituiert ist, (C₅-C₈)-Cycloalkenyl, Phenyl-(C₁-C₄)-alkyl, das im Phenylrest unsubstituiert oder durch einen oder mehrere Reste aus der Gruppe Halogen, (C₁-C₄)-Alkyl, (C₁-C₄)-Alkoxy, (C₁-C₄)-Haloalkyl, (C₁-C₄)-Alkylthio, [(C₁-C₄)-Alkoxy]-carbonyl, [(C₁-C₄)-Alkyl]-carbonyloxy, Carbamoyl, [(C₁-C₄)-Alkyl]-carbonylamino, [(C₁-C₄)-Alkyl]-aminocarbonyl, Di-[(C₁-C₄)-alkyl]-aminocarbonyl und Nitro substituiert ist, oder einen Rest der Formeln A-1 bis A-10 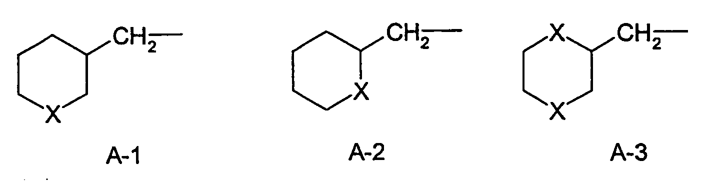 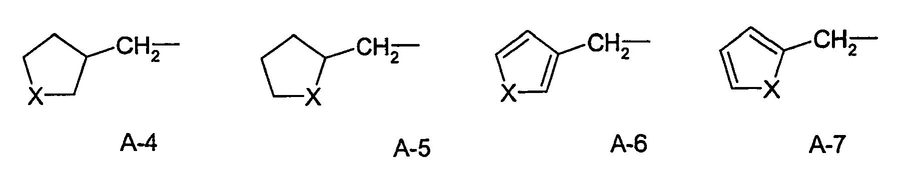 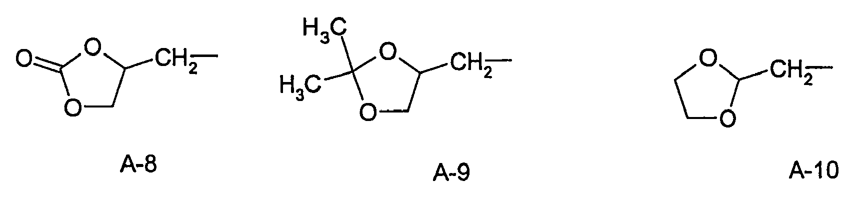 wobei in den Formeln A-1 bis A-10
X oder die X unabhängig voneinander O, S, S(O) oder SO₂ bedeuten,
R¹ Wasserstoff oder (C₁-C₃)-Alkyl,
R² Wasserstoff, Halogen, (C₁-C₃)-Alkyl oder (C₁-C₃)-Alkoxy, wobei jeder der beiden letztgenannten Reste unsubstituiert oder ein- oder mehrfach durch Halogen oder (C₁-C₃)-Alkoxy substituiert ist,
R³ Wasserstoff, Halogen, (C₁-C₃)-Alkyl, (C₁-C₃)-Alkoxy oder (C₁-C₃)-Alkylthio, wobei jeder der letztgenannten drei Reste unsubstituiert oder ein- oder mehrfach durch Halogen oder ein- oder zweifach durch (C₁-C₃)-Alkoxy oder (C₁-C₃)-Alkylthlo substituiert ist, oder einen Rest der Formel NR⁴R⁵, (C₃-C₆)-Cycloalkyl, (C₂-C₄)-Alkenyl, (C₂-C₄)Alkinyl, (C₃-C₄)-Alkenyloxy oder (C₃-C₄)-Alkinyloxy,
R⁴ und R⁵ unabhängig voneinander Wasserstoff, (C₁-C₄)-Alkyl, (C₃-C₄)-Alkenyl, (C₁-C₄)-Haloalkyl oder (C₁-C₄)-Alkoxy
bedeuten,
**dadurch gekennzeichnet, dass** man
a) eine Verbindung der Formel (II) 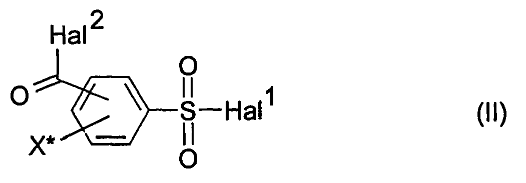 worin
Hal¹ ein Halogenatom bedeutet,
Hal² ein Halogenatom bedeutet, und
X* wie in Formel (I) definiert ist,
durch Reaktion mit einer Verbindung der Formel R-Q-H oder einem Salz davon zur Verbindung der Formel (III) umsetzt, 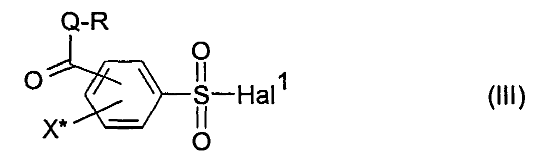 wobei R, Q und X wie in Formel (I) und Hal¹ wie in Formel (II) definiert sind, und
(b) mit oder ohne Zwischenisolierung entweder
(b1) die erhaltene Verbindung (III) zum Sulfonamid der Formel (IV) ammonolysiert, 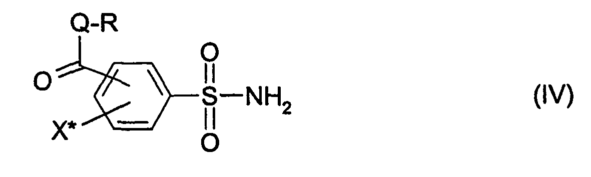 worin R, Q und X* wie in Formel (III) definiert sind,
und die Verbindung (IV) mit oder ohne Zwischenisolierung mit Phosgen zum Phenylsulfonylisocyanat der Formel (V) umsetzt, 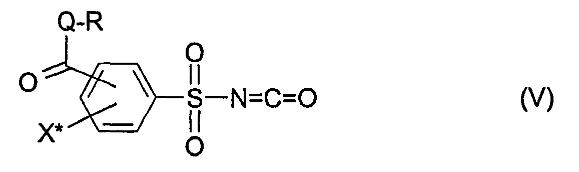 worin R, Q und X* wie in Formel (III) definiert sind,
oder
(b2) die erhaltene Verbindung (III) mit einem Cyanat zu dem Isocyanat der Formel (V) oder einem solvatisierten (stabilisierten) Derivat davon umsetzt, und
(c) mit oder vorzugsweise ohne Zwischenisolierung das Isocyanat der Formel (V) oder dessen stabilisiertes Derivat mit einem heterocyclischen Amin der Formel (VI) 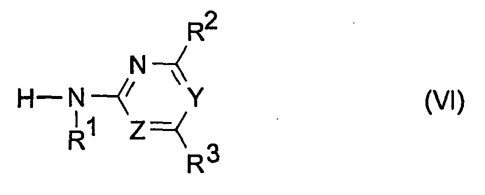 worin R¹, R², R³, Y und Z wie in Formel (I) definiert sind,
zum Sulfonylharnstoff der Formel (I) oder einem Salz davon umsetzt.

2. Verfahren nach Anspruch 1, **dadurch gekennzeichnet, dass** in der Verbindung der Formel (I) oder deren Salzen
Q ein Sauerstoffatom,
X* ein Wasserstoffatom oder Halogenatom,
R (C₁-C₄)-Alkyl, (C₂-C₄)-Alkenyl, (C₂-C₄)-Alkinyl, (C₁-C₄)-Haloalkyl oder (C₁-C₄)-Alkoxy(C₁-C₄)alkyl,
R¹ ein Wasserstoffatom,
R² (C₁-C₄)-Alkyl oder (C₁-C₄)-Alkoxy,
R³ (C₁-C₄)-Alkyl oder (C₁-C₄)-Alkoxy,
Y ein Stickstoffatom oder eine Gruppe der Formel CH und
Z ein Stickstoffatom
bedeuten.

3. Verfahren nach Anspruch 1 oder 2, **dadurch gekennzeichnet, dass** man in Stufe a) Verbindungen der Formel (IIa) als Verbindungen der Formel (II) einsetzt: 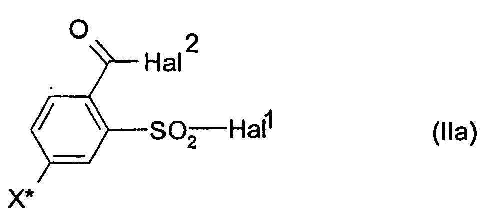 worin
Hal¹ Halogen bedeutet,
Hal² Halogen bedeutet, und
X* wie in Formel (I) definiert ist.

4. Verfahren nach einem der Ansprüche 1 bis 3, **dadurch gekennzeichnet, dass** in der Verbindung der Formel (I) oder deren Salzen
X* ein Iodatom,
R Methyl oder Ethyl,
R² Methoxy,
R³ Methyl und
Y ein Stickstoffatom
bedeuten.

5. Verfahren nach einem der Ansprüche 1 bis 4, **dadurch gekennzeichnet, dass** die Veresterung zum Monoester (III) in einem inerten organischen Lösungsmittel aus der Gruppe der unpolaren aprotischen organischen Lösungsmittel bei einer Temperatur von -20 °C bis 100 °C durchgeführt wird.

6. Verfahren nach einem der Ansprüche 1 bis 5, **dadurch gekennzeichnet, dass** die Veresterung zum Monoester (III) mit einem (C₁-C₄)-Alkanol bei einer Temperatur von -10 °C bis 70 °C oder mit einem Alkalimetall-(C₁-C₄)-alkanolat bei einer Temperatur von -20 °C bis 50 °C durchgeführt wird.

7. Verfahren nach einem der Ansprüche 1 bis 6, **dadurch gekennzeichnet, dass** die Herstellung des Isocyanats (V) in Gegenwart eines aprotischen polaren Lösungmittels bei einer Temperatur von -30 °C bis 70 °C durchgeführt wird.

8. Verfahren nach Anspruch 7, **dadurch gekennzeichnet, dass** die Herstellung des Isocyanats (V) in Gegenwart eines N-Heteroaromaten durchgeführt wird.

9. Verbindungen der Formel (VIII), 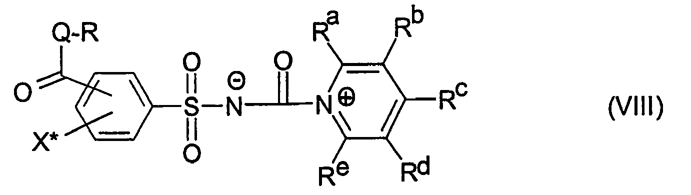 worin R, Q und X* wie in Formel (I) nach Anspruch 1 definiert sind und R^{a}, R^{b}, R^{c}, R^{d} und R^{e} jeweils unabhängig voneinander Wasserstoff, (C₁-C₆)Alkyl, (C₂-C₆)Alkenyl, (C₂-C₆)Alkinyl oder (C₁-C₆)Alkoxy bedeuten oder zwei benachbarte Reste gemeinsam mit den sie verbindenenden C-Atomen des ersten Ringes einen ankondensierten carbocyclischen Ring mit 4 bis 8 C-Atomen oder einen heterocyclischen Ring mit 4 bis 8 Ringatomen und 1, 2 oder 3 Heteroringatomen aus der Gruppe N, O und S bilden.

10. Verfahren zur Herstellung von Verbindungen der Formel (VIII), wie sie in Anspruch 9 definiert sind, **dadurch gekennzeichnet, dass** man eine Verbindung der Formel (III), 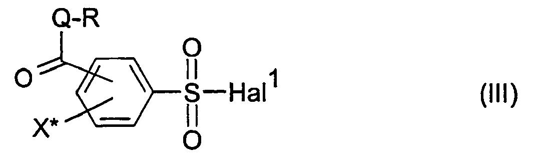 wobei Hal¹ ein Halogenatom bedeutet,
mit einem Cyanat in Gegenwart einer Verbindung der Formel (VII), 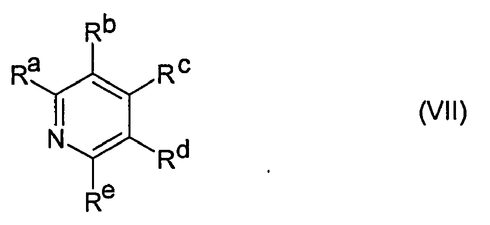 umsetzt,
wobei in den Formel (III) und (VII) die Reste R, R^{a}, R^{b}, R^{c}, R^{d}, R^{e}, Q und X* wie in Formel (VIII) definiert sind.

11. Verfahren zur Herstellung von Verbindungen der Formel (II) 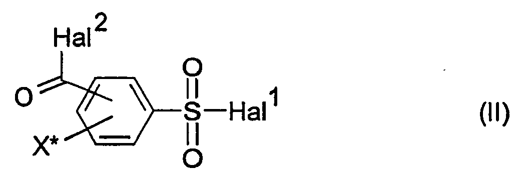 worin Hal¹ und Hal² unabhängig voneinander jeweils ein Halogenatom bedeutet und X* Wasserstoff, Halogen, Cyano, Nitro, (C₁-C₃)-Alkyl oder Methoxy bedeutet, **dadurch gekennzeichnet, dass** man eine Verbindung der Formel (IX) oder ein Salz davon, 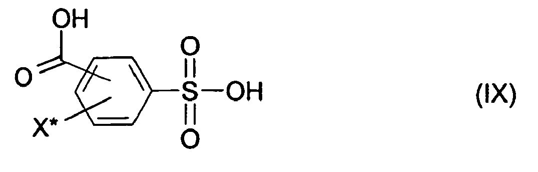 worin X* wie in Formel (II) definiert ist,
mit einem oder mehreren Halogenierungsmitteln aus der Gruppe der anorganischen Säurehalogenide des Schwefels oder Phosphors in einer oder mehreren Reaktionsschritten In Gegenwart eines Katalysators aus der Gruppe der sterisch gehinderten Aminbasen zur Verbindung der Formel (II) umsetzt.

12. Verfahren nach Anspruch 11, **dadurch gekennzeichnet, dass** als Halogenierungsmittel Thionylfluorid, Thionylchlorid, Sulfurylchlorid, Phosphortrichlorid, Phosphorylchlorid, Phosphorpentachlorid oder Phosphortribromid eingesetzt wird.

13. Verfahren nach Anspruch 11 oder 12, **dadurch gekennzeichnet, dass** es in Gegenwart eines inerten organischen Lösungsmittels durchgeführt wird.

14. Verfahren nach einem der Ansprüche 11 bis 13, **dadurch gekennzeichnet, dass** die Reaktionstemperatur im Bereich von 20 °C bis 150 °C liegt.

15. Verbindungen der Formel (IIa), 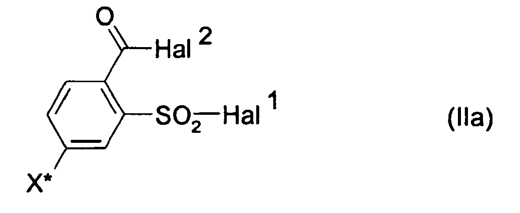 worin Hal¹, Hal² unabhängig voneinander jeweils ein Halogenatom bedeuten und X* ein Iodatom bedeutet.

16. Verbindungen nach Anspruch 15, **dadurch gekennzeichnet, dass** Hal¹ und Hal² jeweils ein Chloratom bedeuten und X* ein Iodatom bedeutet.

17. Verfahren zur Herstellung einer Verbindung der Formel (I) oder deren Salz, wie sie nach Anspruch 1 definiert ist, **dadurch gekennzeichnet, dass** man eine Verbindung der Formel (III), 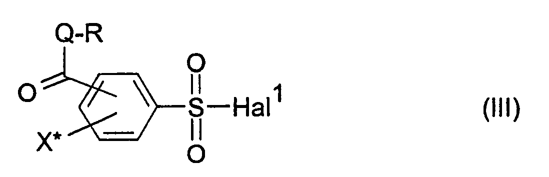 worin R und Q wie in Formel (I) definiert sind und X* wie in Formel (I) definiert ist, mit einem Cyanat zu dem Isocyanat der Formel (V) 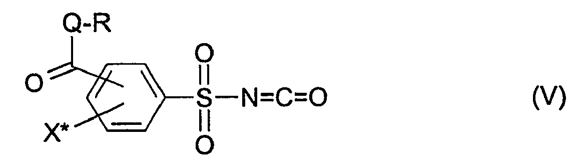 worin R, Q und X* wie in Formel (III) definiert sind,
oder einem solvatisierten (stabilisierten) Derivat davon umsetzt,
und
die erhaltene Verbindung (V) oder dessen stabilisiertes Derivat mit einem heterocyclischen Amin der Formel (VI) 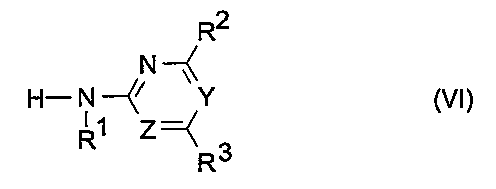 worin R¹, R², R³, Y und Z wie in Formel (I) definiert sind,
zum Sulfonylharnstoff der Formel (I) oder einem Salz davon umsetzt.

## Claims

1. A process for preparing the compound of the formula (I) or a salt thereof 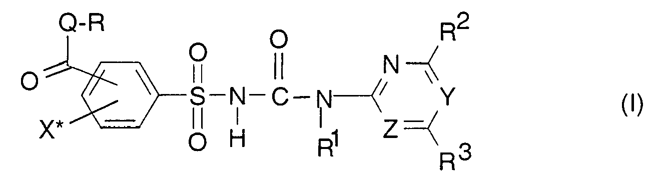 where
Q is oxygen or sulfur,
X* is hydrogen, halogen, cyano, nitro, (C₁-C₃)-alkyl or methoxy,
Y,Z independently of one another are CH or N, where Y and Z are not simultaneously CH,
R is hydrogen, (C₁-C₁₂)-alkyl, (C₂-C₁₀)-alkenyl, (C₂-C₁₀)-alkynyl, (C₁-C₆)-alkyl which is mono- to tetrasubstituted by radicals selected from the group consisting of halogen, (C₁-C₄)-alkoxy, (C₁-C₄)-alkylthio, CN, [(C₁-C₄)-alkoxy]carbonyl and (C₂-C₆)-alkenyl, or (C₃-C₈)-cycloalkyl which is unsubstituted or substituted by radicals selected from the group consisting of (C₁-C₄)-alkyl, (C₁-C₄)-alkoxy, (C₁-C₄)-alkylthio and halogen, (C₅-C₈)-cycloalkenyl, phenyl-(C₁-C₄)-alkyl which is unsubstituted in the phenyl radical or substituted by one or more radicals selected from the group consisting of halogen, (C₁-C₄)-alkyl, (C₁-C₄)-alkoxy, (C₁-C₄)-haloalkyl, (C₁-C₄)-alkylthio, [(C₁-C₄)-alkoxy]carbonyl, [(C₁-C₄)-alkyl]carbonyloxy, carbamoyl, [(C₁-C₄)-alkyl]carbonylamino, [(C₁-C₄)-alkyl]aminocarbonyl, di-[(C₁-C₄)-alkyl]aminocarbonyl and nitro, or a radical of the formulae A-1 to A-10 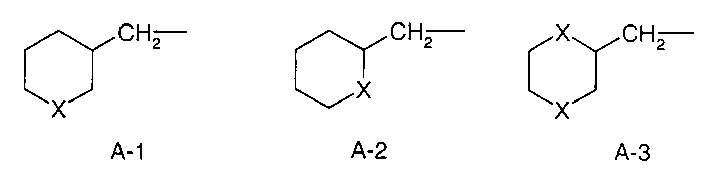 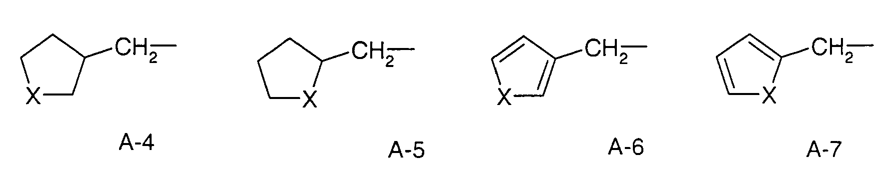 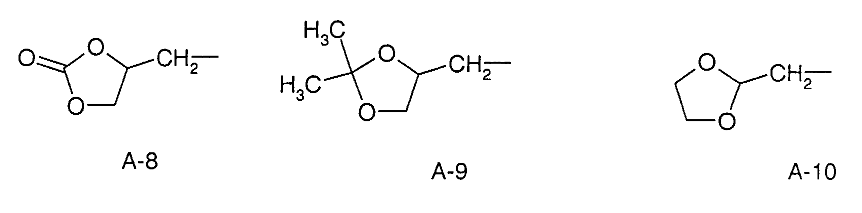 where in the formulae A-1 to A-1 0
the radical X or the radicals X independently of one another is/are O, S, S(O) or SO₂,
R¹ is hydrogen or (C₁-C₃)-alkyl,
R² is hydrogen, halogen, (C₁-C₃)-alkyl or (C₁-C₃)-alkoxy, where each of the two last-mentioned radicals is unsubstituted or mono- or polysubstituted by halogen or (C₁-C₃)-alkoxy,
R³ is hydrogen, halogen, (C₁-C₃)-alkyl, (C₁-C₃)-alkoxy or (C₁-C₃)-alkylthio, where each of the three last-mentioned radicals is unsubstituted or mono- or polysubstituted by halogen or mono- or disubstituted by (C₁-C₃)-alkoxy or (C₁-C₃)-alkylthio, or a radical of the formula NR⁴R⁵, (C₃-C₆)-cycloalkyl, (C₂-C₄)-alkenyl, (C₂-C₄)-alkynyl, (C₃-C₄)-alkenyloxy or (C₃-C₄)-alkynyloxy,
R⁴ and R⁵ independently of one another are hydrogen, (C₁-C₄)-alkyl, (C₃-C₄)-alkenyl, (C₁-C₄)-haloalkyl or (C₁-C₄)-alkoxy,
which comprises
a) converting a compound of the formula (II) 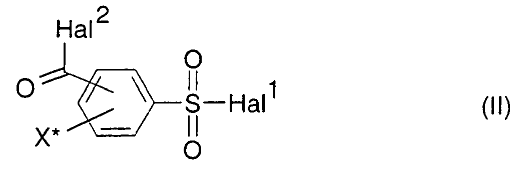 where
Hal¹ is a halogen atom,
Hal² is a halogen atom and
X* is as defined in formula (I)
by reaction with a compound of the formula R-Q-H or a salt thereof into the compound of the formula (III) 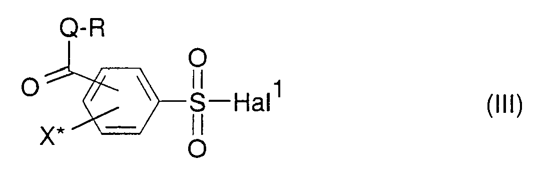 where R, Q and X are as defined in formula (I) and Hal¹ is as defined in formula (II), and
(b) with or without intermediate isolation either
(b1) ammonolyzing the resulting compound (III) to give the sulfonamide of the formula (IV) 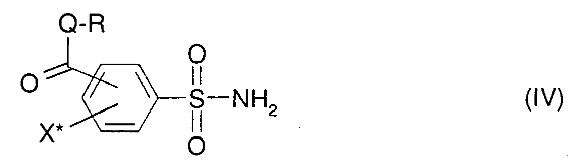 where R, Q and X* are as defined in formula (III),
and converting the compound (IV) with or without intermediate isolation with phosgene into the phenylsulfonyl isocyanate of the formula (V) 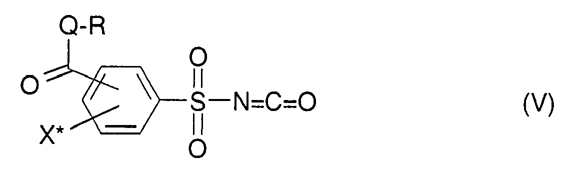 where R, Q and X* are as defined in formula (III),
or
(b2)converting the resulting compound (III) with a cyanate into the isocyanate of the formula (V) or a solvated (stabilized) derivative thereof,
and
(c) converting the isocyanate of the formula (V) or its stabilized derivative, with or preferably without intermediate isolation, with a heterocyclic amine of the formula (VI) 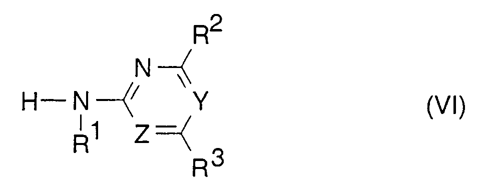 where R¹, R², R³, Y and Z are as defined in formula (I),
into the sulfonylurea of the formula (I) or a salt thereof.

2. The process as claimed in claim 1, wherein in the compound of the formula (I) or its salt
Q is an oxygen atom,
X* is a hydrogen atom or a halogen atom,
R is (C₁-C₄)-alkyl, (C₂-C₄)-alkenyl, (C₂-C₄)-alkynyl, (C₁-C₄)-haloalkyl or (C₁-C₄)-alkoxy(C₁-C₄)-alkyl,
R¹ is a hydrogen atom,
R² is (C₁-C₄)-alkyl or (C₁-C₄)-alkoxy,
R³ is (C₁-C₄)-alkyl or (C₁-C₄)-alkoxy,
Y is a nitrogen atom or a group of the formula CH and
Z is a nitrogen atom.

3. The process as claimed in claim 1 or 2, wherein in step a) the compounds of the formula (II) used are compounds of the formula (Ila): 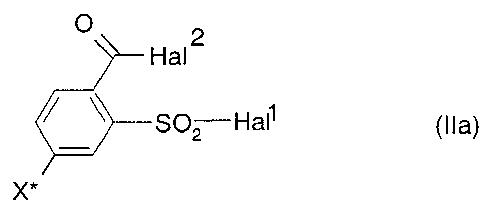 where
Hal¹ is halogen,
Hal² is halogen, and
X* is as defined in formula (I).

4. The process as claimed in any of claims 1 to 3, wherein in the compound of the formula (I) or its salt
X* is an iodine atom,
R is methyl or ethyl,
R² is methoxy,
R³ is methyl and
Y is a nitrogen atom.

5. The process as claimed in any of claims 1 to 4, wherein the esterification
to the monoester (III) is carried out in an inert organic solvent selected from the group of the nonpolar aprotic organic solvents, at a temperature of from -20°C to 100°C.

6. The process as claimed in any of claims 1 to 5, wherein the esterification
to the monoester (III) is carried out using a (C₁-C₄)-alkanol at a temperature of from -10°C to 70°C or using an alkali metal (C₁-C₄)-alkoxide at a temperature of from -20°C to 50°C.

7. The process as claimed in any of claims 1 to 6, wherein the preparation
of the isocyanate (V) is carried out in the presence of an aprotic polar solvent at a temperature of from -30°C to 70°C.

8. The process as claimed in claim 7, wherein the preparation
of the isocyanate (V) is carried out in the presence of an N-heteroaromatic compound.

9. A compound of the formula (VIII) 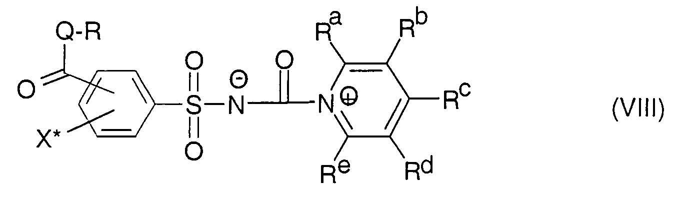 where R, Q and X* are as defined in formula (I) as set forth in claim 1 and R^{a}, R^{b}, R^{c}, R^{d} and R^{e} are each independently of one another hydrogen, (C₁-C₆)-alkyl, (C₂-C₆)-alkenyl, (C₂-C₆)-alkynyl or (C₁-C₆)-alkoxy or two adjacent radicals together with the linking carbon atoms of the first ring form a fused-on carbocyclic ring having 4 to 8 carbon atoms or a heterocyclic ring having 4 to 8 ring atoms and 1, 2 or 3 heteroring atoms selected from the group consisting of N, O and S.

10. A process for preparing compounds of the formula (VIII) as defined in claim 9, which comprises reacting a compound of the formula (III) 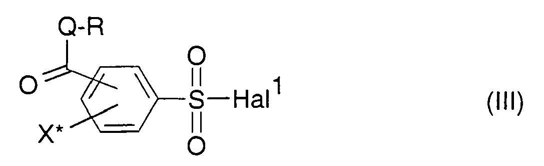 where Hal¹ is a halogen atom
with a cyanate in the presence of a compound of the formula (VII) 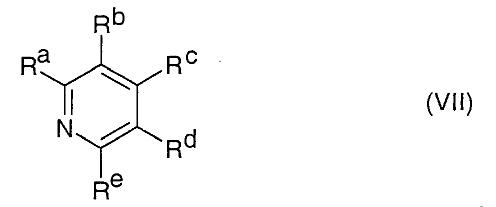 where in the formulae (III) and (VII) the radicals R, R^{a}, R^{b}, R^{c}, R^{d}, R^{e}, Q and X* are as defined in formula (VIII).

11. A process for preparing compounds of the formula (II) 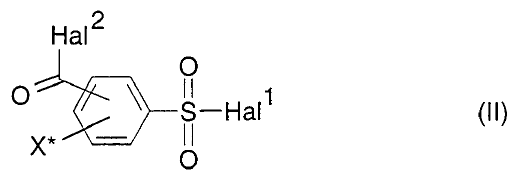 where Hal¹ and Hal² are each independently of one another a halogen atom and X* is hydrogen, halogen, cyano, nitro, (C₁-C₃)-alkyl or methoxy, which comprises converting a compound of the formula (IX) or a salt thereof 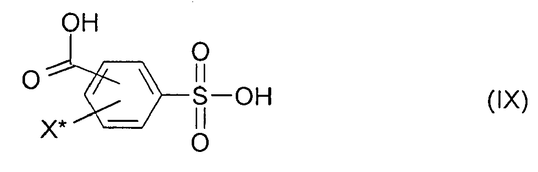 where X* is as defined in formula (II)
with one or more halogenating agents selected from the group of the inorganic acid halides of sulfur or phosphorus, in one or more reaction steps in the presence of a catalyst selected from the group of the sterically hindered amine bases, into the compound of the formula (II).

12. The process as claimed in claim 11, wherein the halogenating agent used is thionyl fluoride, thionyl chloride, sulfuryl chloride, phosphorus trichloride, phosphoryl chloride, phosphorus pentachloride or phosphorus tribromide.

13. The process as claimed in claim 11 or 12, wherein the process is carried out in the presence of an inert organic solvent.

14. The process as claimed in any of claims 11 to 13, wherein the reaction temperature is in the range from 20°C to 150°C.

15. A compound of the formula (IIa) 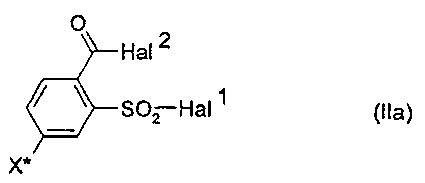 where Hal¹, Hal² are each independently of one another a halogen atom and X* is an iodine atom.

16. The compound as claimed in claim 15, wherein Hal¹ and Hal² are each a chlorine atom and X* is an iodine atom.

17. A process for preparing a compound of the formula (I), or a salt thereof, as defined in claim 1, wherein a compound of the formula (III) 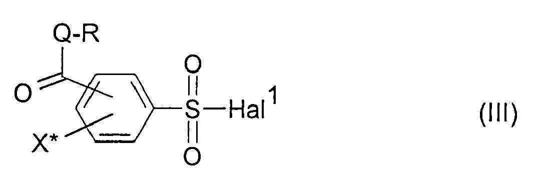 where R and Q are as defined in formula (I) and X* is as defined in formula (I), is converted with a cyanate into the isocyanate of the formula (V) 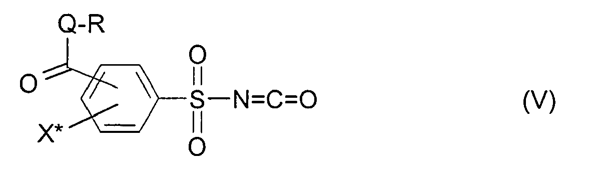 where R, Q and X* are as defined in formula (III),
or a solvated (stabilized) derivative thereof,
and
the resulting compound (V) or its stabilized derivative is converted with a heterocyclic amine of the formula (VI) 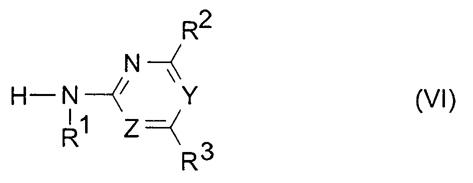 where R¹, R², R³, Y and Z are as defined in formula (I),
into the sulfonylurea of the formula (I) or a salt thereof.

## Revendications

1. Procédé pour la préparation du composé de formule (I) ou de ses sels 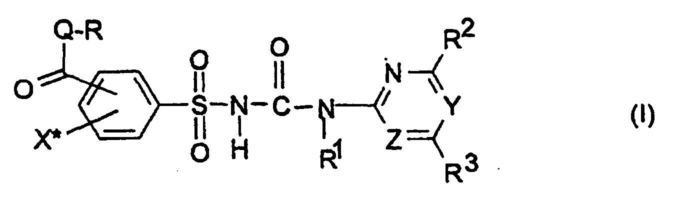 où
Q représente un atome d'oxygène ou de soufre,
X* représente un atome d'hydrogène, un atome d'halogène, un groupe cyano, nitro, alkyle en C₁-C₃ ou méthoxy,
Y, Z indépendamment l'un de l'autre, représentent CH ou N, Y et Z ne sont pas simultanément CH,
R représente un atome d'hydrogène, un groupe alkyle en C₁-C₁₂, alcényle en C₂-C₁₀, alcynyle en C₂-C₁₀, alkyle en C₁-C₆ qui est substitué une à quatre fois par des restes pris dans le groupe constitué par un halogène, alkoxy en C₁-C₄, (alkyl en C₁-C₄)thio, CN, (alkoxy en C₁-C₄)-carbonyle et alcényle en C₂-C₆, ou cycloalkyle en C₃-C₈, qui est non substitué ou substitué par des restes pris dans le groupe constitué par un alkyle en C₁-C₄, alkoxy en C₁-C₄, (alkyl en C₁-C₄)thio et halogène, cycloalcényle en C₅-C₈, phénylalkyle en C₁-C₄, qui est non substitué ou substitué sur le reste phényle par un ou plusieurs restes pris dans le groupe constitué par un halogène, alkyle en C₁-C₄, alkoxy en C₁-C₄, haloalkyle en C₁-C₄, (alkyl en C₁-C₄)thio, (alkoxy en C₁-C₄)-carbonyle, (alkyl en C₁-C₄)carbonyloxy, carbamoyle, (alkyl en C₁-C₄)-carbonylamino, (alkyl en C₁-C₄)-aminocarbonyle, di(alkyl en C₁-C₄)-aminocarbonyle et nitro, ou un reste de formules A-1 à A-10 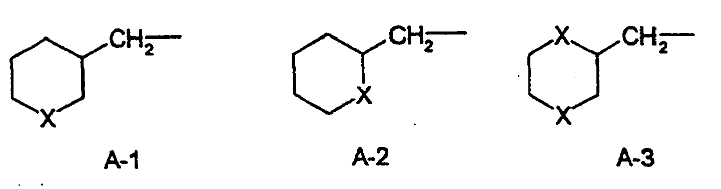 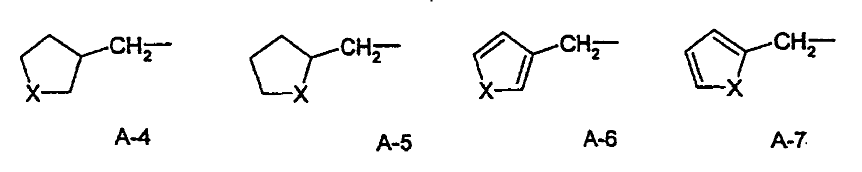 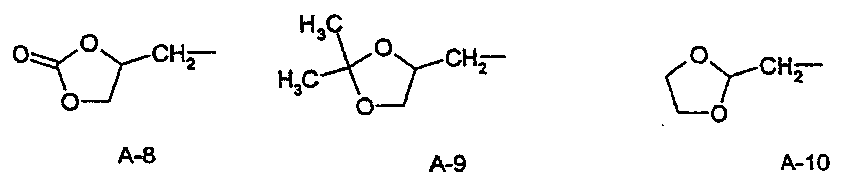 où dans les formules A-1 à A-10
X ou des X, indépendamment les uns des autres, représentent O, S, S(O) ou SO₂
R¹ représente un atome d'hydrogène ou un groupe alkyle en C₁-C₃,
R² représente un atome d'hydrogène, un atome d'halogène, un groupe alkyle en C₁-C₃ ou alkoxy en C₁-C₃, chacun des trois derniers restes cités étant non substitué ou substitué une ou plusieurs fois par un substituant halogène ou alkoxy en C₁-C₃,
R³ représente un atome d'hydrogène, un atome d'halogène, un groupe alkyle en C₁-C₃, alkoxy en C₁-C₃ ou (alkyl en C₁-C₃)thio, chacun des trois derniers restes cités étant non substitué ou substitué une ou plusieurs fois par un halogène ou une ou deux fois par un alkoxy en C₁-C₃ ou (alkyl en C₁-C₃)thio, ou un reste de formule NR⁴R⁵, cycloalkyle en C₃-C₆, alcényle en C₂-C₄, alcynyle en C₂-C₄, (alcényl en C₃-C₄)oxy ou (alcynyl en C₃-C₄)oxy,
R⁴ et R⁵, indépendamment l'un de l'autre, représentent un atome d'hydrogène, un groupe alkyle en C₁-C₄, alcényle en C₃-C₄, haloalkyle en C₁-C₄ ou alkoxy en C₁-C₄,
**caractérisé en ce qu'**on fait réagir
a) un composé de formule (II) 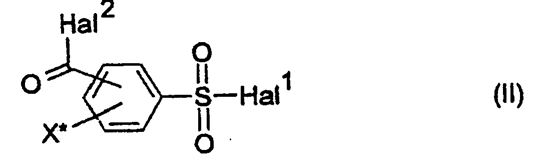 dans laquelle
Hal¹ représente un atome d'halogène,
Hal² représente un atome d'halogène, et
X* est défini comme dans la formule (I),
par réaction sur un composé de formule R-Q-H ou un sel de celui-ci, en composé de formule (III) 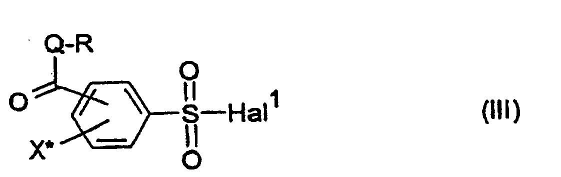 où R, Q et X sont définis comme dans la formule (I) et Hall comme dans la formule (II), et
b) avec ou sans isolement intermédiaire, soit
b1) le composé (III) obtenu est ammonolysé en sulfonamide de formule (IV), 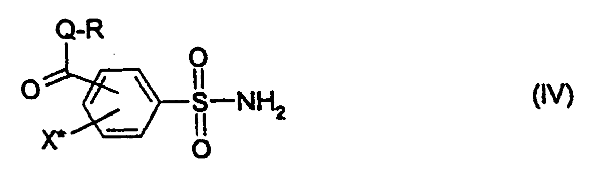 où R, Q et X* sont définis comme dans la formule (III),
et on fait réagir le composé (IV) avec ou sans isolement intermédiaire sur du phosgène en isocyanate de phénylsulfonyle de formule (V), 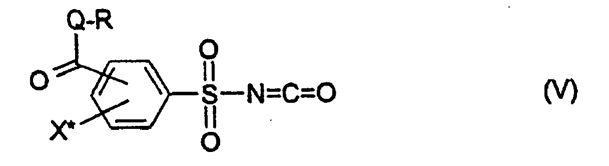 où R, Q et X* sont définis comme dans la formule (III),
ou
(b2) on fait réagir le composé (III) obtenu sur un cyanate en l'isocyanate de formule (V) ou un dérivé solvaté (stabilisé) de celui-ci,
et
(c) on fait réagir avec ou de préférence sans isolement intermédiaire l'isocyanate de formule (V) ou son dérivé stabilisé sur une amine hétérocyclique de formule (VI) 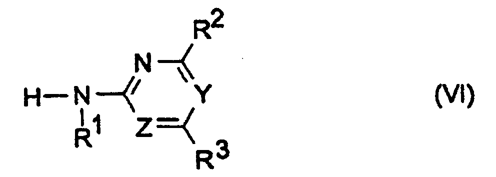 où R¹, R², R³, Y et Z sont définis comme dans la formule (I),
en sulfonylurée de formule (I) ou un sel de celle-ci.

2. Procédé selon la revendication 1, **caractérisé en ce que** dans le composé de formule (I) ou ses sels représentent
Q un atome d'oxygène,
X* un atome d'hydrogène ou un atome d'halogène,
R un groupe alkyle en C₁-C₄, alcényle en C₂-C₄, alcynyle en C₂-C₄, haloalkyle en C₁-C₄ ou alkoxyalkyle en C₁-C₄,
R¹ un atome d'hydrogène,
R² un groupe alkyle en C₁-C₄ ou alkoxy en C₁-C₄,
R³ un groupe alkyle en C₁-C₄ ou alkoxy en C₁-C₄,
Y un atome d'azote ou un groupe de formule CH et
Z un atome d'azote.

3. Procédé selon la revendication 1 ou 2, **caractérisé en ce que** dans l'étape a), on utilise des composés de formule (IIa) en tant que composés de formule (II) : 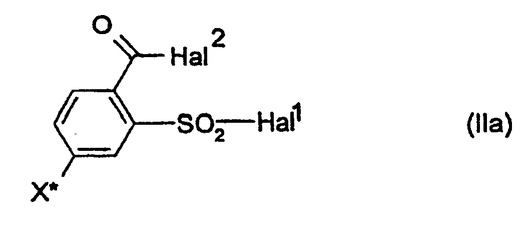 où
Hall représente un atome d'halogène,
Hal² représente un atome d'halogène, et
X* est défini comme dans la formule (I).

4. Procédé selon l'une des revendications 1 à 3, **caractérisé en ce que** dans le composé de formule (I) ou ses sels représentent
X* un atome d'iode,
R un groupe méthyle ou éthyle,
R² un groupe méthoxy,
R³ un groupe méthyle et
Y un atome d'azote.

5. Procédé selon l'une des revendications 1 à 4, **caractérisé en ce que** l'estérification en monoester (III) est mise en oeuvre dans un solvant organique inerte pris dans le groupe des solvants organiques non polaires aprotiques, à une température de -20°C à 100°C.

6. Procédé selon l'une des revendications 1 à 5, **caractérisé en ce que** l'estérification en monoester (III) est mise en oeuvre avec un alcanol en C₁-C₄ à une température de -10°C à 70°C, ou avec un alcanolate en C₁-C₄ de métal alcalin à une température de -20°C à 50°C.

7. Procédé selon l'une des revendications 1 à 6, **caractérisé en ce que** la préparation de l'isocyanate (V) est mise en oeuvre en présence d'un solvant aprotique polaire, à une température de -30°C à 70 C.

8. Procédé selon la revendication 7, **caractérisé en ce que** la préparation de l'isocyanate (V) est mise en oeuvre en présence d'un hétéroatome N.

9. Composés de formule (VIII) 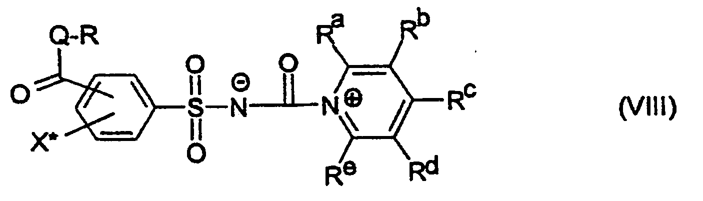 où R, Q et X* sont définis comme dans la formule (I) selon la revendication 1 et R^{a}, R^{b}, R^{c}, R^{d} et R^{e} chacun, indépendamment l'un de l'autre, représentent un atome d' hydrogène, un groupe alkyle en C₁-C₆, alcényle en C₂-C₆, alcynyle en C₂-C₆ ou alkoxy en C₁-C₆, ou deux restes voisins, conjointement avec les atomes de carbone du premier cycle qui les relient, forment un cycle carbocyclique condensé à 4 à 8 atomes de carbone ou un cycle hétérocyclique à 4 à 8 chaînons et 1, 2 ou 3 chaînons hétéroatomes pris dans le groupe comprenant un atome de N, de O et de S.

10. Procédé pour la préparation de composés de formule (VIII), tels qu'ils sont définis dans la revendication 9, **caractérisé en ce qu'**on fait réagir un composé de formule (III), 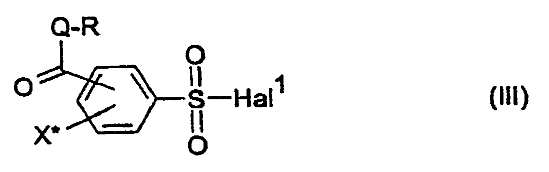 où Hall représente un atome d'halogène,
sur un cyanate en présence d'un composé de formule (VII) 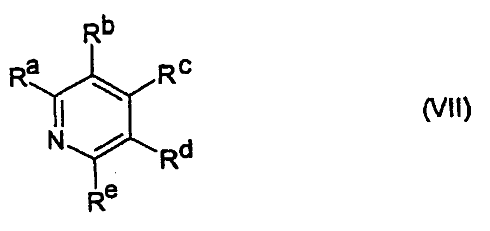 où dans les formules (III) et (VII) les restes R, R^{a}, R^{b}, R^{c}, R^{d}, R^{e}, Q et X* sont définis comme dans la formule (VIII).

11. Procédé pour la préparation de composés de formule (II) 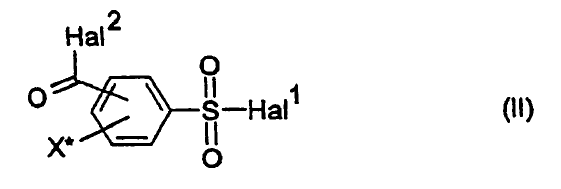 où Hall et Hal² chacun, indépendamment l'un de l'autre, représentent un atome d'halogène, et X* représente un atome d'hydrogène, un atome d'halogène, un groupe cyano, nitro, alkyle en C₁-C₃ ou méthoxy,
**caractérisé en ce qu'**on fait réagir un composé de formule (IX) ou un sel de celui-ci 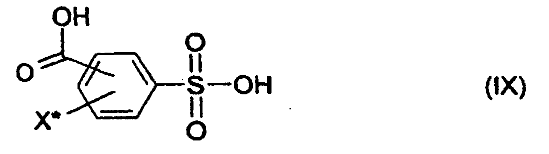 où X* est défini comme dans la formule (II),
sur un ou plusieurs agents d'halogénation pris dans le groupe des halogénures d'acides inorganiques du soufre
ou du phosphore, dans une ou plusieurs étapes réactionnelles, en présence d'un catalyseur pris dans le groupe des bases aminées à encombrement stérique, en composé de formule (II).

12. Procédé selon la revendication 11, **caractérisé en ce qu'**on utilise comme agent d'halogénation le fluorure de thionyle, le chlorure de thionyle, le chlorure de sulfuryle, le trichlorure de phosphore, le chlorure de phosphoryle, le pentachlorure de phosphore ou le tribromure de phosphore.

13. Procédé selon la revendication 11 ou 12, **caractérisé en ce qu'**il est mis en oeuvre en présence d'un solvant organique inerte.

14. Procédé selon l'une des revendication 11 à 13, **caractérisé en ce que** la température réactionnelle se situe dans le domaine de 20°C à 150°C.

15. Composés de formule (IIa) 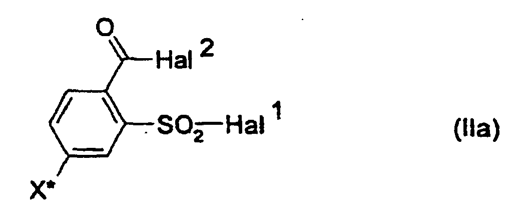 dans laquelle Hal¹, Hal², indépendamment l'un de l'autre, représentent un atome d'halogène et X* représente un atome d'iode.

16. Composés selon la revendication 15,
**caractérisés en ce que** Hall et Hal² chacun représentent un atome de chlore et X* un atome d'iode.

17. Procédé pour la préparation d'un composé de formule (I) ou son sel comme défini dans la revendication 1, **caractérisé en ce qu'**on fait réagir un composé de formule (III) 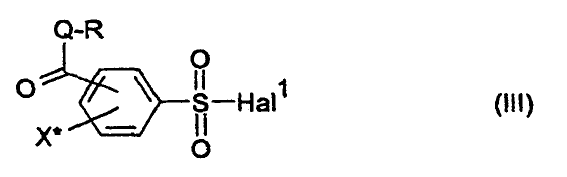 où R et Q sont définis comme dans la formule (I) et X* est défini comme dans la formule (I), sur un cyanate en l'isocyanate de formule (V) 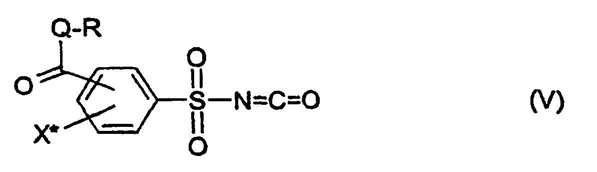 où R, Q et X* sont définis comme dans la formule (III),
ou un dérivé solvaté (stabilisé) de celui-ci,
et
on fait réagir le composé (V) obtenu ou son dérivé stabilisé sur une amine hétérocyclique de formule (VI) 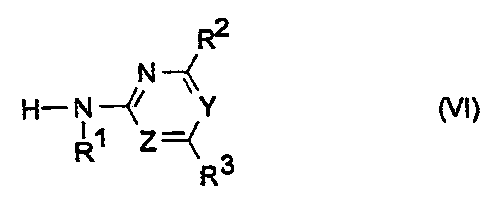 où R¹, R², R³, Y et Z dont définis comme dans la formule (I),
en sulfonylurée de formule (I) ou un sel de celle-ci.
